# EUROPÄISCHE PATENTSCHRIFT

(11) **EP 1 073 641 B1**
(45) Veröffentlichungstag und Bekanntmachung des Hinweises auf die Patenterteilung: **14.04.2004**
(21) Anmeldenummer: 99922108.8
(22) Anmeldetag: 20.04.1999
(51) Int. Cl.: C07D 237/36, C07D 237/32, C07D 213/82, C07D 295/155, C07D 209/14, C07D 233/64, A61K 31/16, A61K 31/395, C07C 237/20, C07C 237/22

(54) **NEUE SUBSTITUIERTE AMIDE, DEREN HERSTELLUNG UND ANWENDUNG**
NEW SUBSTITUTED AMIDES, THEIR PRODUCTION AND THEIR USE
INHIBITEURS DE PROTEASE DE CYSTEINE

(30) Priorität: 20.04.1998 DE 19818615
(43) Veröffentlichungstag der Anmeldung: 07.02.2001
(73) Patentinhaber: Abbott GmbH & Co. KG, 65205 Wiesbaden (DE)
(72) Erfinder: LUBISCH, Wilfried, D-69115 Heidelberg (DE); MÖLLER, Achim, D-67269 Grünstadt (DE); TREIBER, Hans-Jörg, D-68782 Brühl (DE); KNOPP, Monika, D-67071 Ludwigshafen (DE)
(74) Vertreter: Grünecker, Kinkeldey, Stockmair & Schwanhäusser Anwaltssozietät
(86) Internationale Anmeldenummer: PCT/EP1999/002633
(87) Internationale Veröffentlichungsnummer: WO 1999/054310

(56) Entgegenhaltungen:
- EP-A- 0 520 336
- DE-A- 19 642 591

## Beschreibung

Die vorliegende Erfindung betrifft neuartige Amide, die Inhibitoren von Enzymen, insbesondere Cystein-Proteasen, wie Calpain (= Calcium dependant cysteine proteases) und dessen Isoenzyme und Cathepsine, zum Beispiel B und L, darstellen.

Calpaine stellen intracelluläre, proteolytische Enzyme aus der Gruppe der sogenannten Cystein-Proteasen dar und werden in vielen Zellen gefunden. Calpaine werden durch erhöhte Kalziumkonzentration aktiviert, wobei man zwischen Calpain I oder µ-Calpain, das durch µ-molare Konzentrationen von Calzium-Ionen aktiviert wird, und Calpain II oder m-Calpain, das durch m-molare Konzentrationen von Kalzium-Ionen aktiviert wird, unterscheidet (P. Johnson, Int. J. Biochem. 1990, *22(8)*, 811-22). Heute werden noch weitere Calpain-Isoenzyme postuliert (K. Suzuki et al., *Biol. Chem. Hoppe-Seyler,* 1995, *376(9)*,523-9).

Man vermutet, daß Calpaine in verschiedenen physiologischen Prozessen eine wichtige Rolle spielen. Dazu gehören Spaltungen von regulatorischen Proteinen wie Protein-Kinase C, Cytoskelett-Proteine wie MAP 2 und Spektrin, Muskelproteine, Proteinabbau in rheumatoider Arthritis, Proteine bei der Aktivierung von Plättchen, Neuropeptid-Metabolismus, Proteine in der Mitose und weitere, die in. M.J. Barrett et al., *Life Sci*. 1991, *48*, 1659-69 und K.K. Wang et al., *Trends in Pharmacol*. *Sci*., 1994, 15, 412-9, aufgeführt sind.

Bei verschiedenen pathophysiologischen Prozessen wurden erhöhte Calpain-Spiegel gemessen, zum Beispiel: Ischämien des Herzens (z.B. Herzinfarkt), der Niere oder des Zentralnervensystems (z.B. "Stroke"), Entzündungen, Muskeldystrophien, Katarakten der Augen, Verletzungen des Zentralnervensystems (z.B. Trauma), Alzheimer Krankheit usw. (siehe K.K. Wang, oben). Man vermutet einen Zusammenhang dieser Krankheiten mit erhöhten und anhaltenden intrazellulären Kalziumspiegeln. Dadurch werden Kalzium-abhängige Prozesse überaktiviert und unterliegen nicht mehr der physiologischen Regelung. Dementsprechend kann eine Überaktivierung von Calpainen auch pathophysiologische Prozesse auslösen.

Daher wurde postuliert, daß Inhibitoren der Calpain-Enzyme für die Behandlung dieser Krankheiten nützlich sein können. Verschiedene Untersuchungen bestätigen dies. So haben Seung-Chyul Hong et al., *Stroke* 1994, 25(3), 663-9 und R.T. Bartus et al., *Neurological Res.* 1995, *17,* 249-58 eine neuroprotektive Wirkung von Calpain-Inhibitoren in akuten neurodegenerativen Störungen oder Ischämien, wie sie nach Hirnschlag auftreten, gezeigt. Ebenso nach experimentellen Gehirntraumata verbesserten Calpain-Inhibitoren die Erholung der auftretenden Gedächtnisleistungsdefizite und neuromotrischen Störungen (K.E. Saatman et al. *Proc*. *Natl. Acad. Sci. USA*, 1996, *93*, 3428-3433). C.L. Edelstein et al., *Proc. Natl. Acad. Sci. USA*, 1995, *92*, 7662-6, fand eine protektive Wirkung von Calpain-Inhibitoren auf durch Hypoxie geschädigten Nieren. Yoshida, Ken Ischi et al., *Jap. Circ. J.* 1995, *59(1),* 40-8, konnten günstige Effekte von Calpain-Inhibitoren nach cardialen Schädigungen aufzeigen, die durch Ischämie oder Reperfusion erzeugt wurden. Da Calpain-Inhibitoren die Freisetzung von dem β-AP4-Protein hemmen, wurde eine potentielle Anwendung als Therapeutikum der Alzheimer Krankheit vorgeschlagen (J. Higaki et al., *Neuron,* 1995, *14,* 651-59). Die Freisetzung von Interleukin-1α wird ebenfalls durch Calpain-Inhibitoren gehemmt (N. Watanabe et al., *Cytokine* 1994, *6(6),* 597-601). Weiterhin wurde gefunden, daß Calpain-Inhibitoren cytotoxische Effekte an Tumorzellen zeigen ( E. Shiba et al. *20*^{*th*} *Meeting Int. Ass. Breast Cancer Res., Sendai Jp,* 1994, 25.-28.Sept., *Int. J. Oncol*. *5 (Suppl.),* 1994, 381).
Weitere mögliche Anwendungen von Calpain-Inhibitoren sind in K.K. Wang, *Trends* in *Pharmacol. Sci*., 1994, *15,* 412-8, aufgeführt.

Calpain-Inhibitoren sind in der Literatur bereits beschrieben worden. Überwiegend sind dies jedoch entweder irreversible oder peptidische Inhibitoren. Irreversible Inhibitoren sind in der Regel alkylierende Substanzen und haben den Nachteil, daß sie im Organismus unselektiv reagieren oder instabil sind. So zeigen diese Inhibitoren oft unerwünschte Nebeneffekte, wie Toxizität, und sind danach in der Anwendung eingeschränkt oder nicht brauchbar. Zu den irreveriblen Inhibitoren kann man zum Beispiel die Epoxide E 64 (E.B. McGowan et al., *Biochem*. *Biophys*. *Res.Commun*. 1989, 158, 432-5), α-Halogenketone (H. Angliker et al., *J*. *Med. Chem*. 1992, 35, 216-20) oder Disulfide (R. Matsueda et al., *Chem*. *Lett*. 1990, 191-194) zählen.

Viele bekannte reversible Inhibitoren von Cystein-Proteasen wie Calpain stellen peptidische Aldehyde dar, insbesondere dipeptidische und tripepidische Aldehyde wie zum Beispiel Z-Val-Phe-H (MDL 28170) (S. Mehdi, *Trends in Biol*. *Sci*. 1991, 16, 150-3). Unter physiologischen Bedingungen haben peptidische Aldehyde den Nachteil, daß sie auf Grund der großen Reaktivität häufig instabil sind, schnell metabolisiert werden können und zu unspezifischen Reaktionen neigen, die die Ursache von toxischen Effekten sein können (J.A. Fehrentz und B.Castro, *Synthesis* 1983, 676-78).

In JP 08183771 (CA 1996, 605307) und in EP 520336 sind Aldehyde, die sich von 4-Piperidinoylamide und 2-Carbonyl-piperidino-4-ylamide ableiten als Calpain-Inhibitoren beschrieben worden. Jedoch sind die hier beanspruchten Aldehyde, die sich von heteroaromatisch substituierten Amiden der allgemeinen Struktur I ableiten bisher noch beschrieben worden.

Peptidische Keton-Derivate sind ebenfalls Inhibitoren von Cystein-Proteasen, insbesondere Calpaine. So sind zum Beispiel bei Serin-Proteasen Keton-Derivate als Inhibitoren bekannt, wobei die Keto-Gruppe von einer elektronenziehenden Gruppe wie CF₃ aktiviert wird. Bei Cystein-Proteasen sind Derivate mit durch CF₃ oder ähnlichen Gruppen aktivierte Ketone wenig oder nicht wirksam (M.R.Angelastro et al., *J. Med. Chem.* 1990, *33*, 11-13). Überraschenderweise konnten bei Calpain bisher nur Keton-Derivate, bei denen einerseits α-ständige Abgangsgruppen eine irreversible Hemmung verursachen und andererseits ein Carbonsäure-Derivat die Keto-Gruppe aktiviert, als wirksame Inhibitoren gefunden werden (siehe M.R.Angelastro et al., siehe oben; WO 92/11850; WO 92,12140; WO 94/00095 und WO 95/00535). Jedoch sind von diesen Ketoamiden und Ketoestern bisher nur peptidische Derivate als wirksam beschrieben worden (Zhaozhao Li et al., *J*. *Med*. *Chem*. 1993, 36, 3472-80; S.L.Harbenson et al., *J*. *Med*. *Chem*. 1994, 37, 2918-29 und siehe oben M.R. Angelastro et al.).

Ketobenzamide sind bereits in der Literatur bekannt. So wurde der Ketoester PhCO-Abu-COOCH₂CH₃ in WO 91/09801, WO 94/00095 und 92/11850 beschrieben. Das analoge Phenyl-Derivat Ph-CONH-CH(CH₂Ph)-CO-COCOOCH₃ wurde in M.R.Angelastro et al., *J*. *Med*. *Chem*. 1990, 33, 11-13 als jedoch nur schwacher Calpain-Inhibitor gefunden. Dieses Derivat ist auch in J.P. Burkhardt, *Tetrahedron Lett*., 1988, 3433-36 beschrieben. Die Bedeutung der substituierten Benzamide ist jedoch bisher nie untersucht worden.

Nicht-peptidische Piperidin-Ketocarbonsäure-Derivate, welche Inhibitoren von Cystein-Proteasen darstellen, werden in der DE 19642591 A beschrieben.

In einer Reihe von Therapien wie Schlaganfall werden die Wirkstoffe intravenös zum Beispiel als Infusionslösung appliziert. Dazu ist es notwendig Substanzen, hier Calpain-Inhibitoren, zur Verfügung zu haben, die ausreichende Wasserlöslichkeit aufweisen, so daß eine Infusionslösung hergestellt werden kann. Viele der beschriebenen Calpain-Inhibitoren haben jedoch den Nachteil, daß sie nur geringe oder keine Wasserlöslichkeit zeigen und somit nicht für eine intravenöse Applikation in Frage kommen. Derartige Wirkstoffe können nur mit Hilfsstoffen, die die Wasserlöslichkeit vermitteln sollen, appliziert werden (vgl. R.T. Bartus et al. *J. Cereb. Blood Flow Metab*. 1994, *14,* 537-544). Diese Hilfsstoffe, zum Beispiel Polyethylenglykol, haben aber häufig Begleiteffekte oder sind sogar unverträglich. Ein nichtpeptidischer Calpain-Inhibitor, der also ohne Hilfsstoffe wasserlöslich ist, hätte somit einen großen Vorteil. Ein solcher Inhibitor ist bisher nicht beschrieben worden und wäre damit neu.

In der vorliegenden Erfindung wurden nicht-peptidische Aldehyde, Ketocarbonsäureester und Ketoamid-Derivate beschrieben. Diese Verbindungen sind neu und zeigen überraschenderweise die Möglichkeit auf, durch Einbau von rigiden strukturellen Fragmenten potente nicht-peptidische Inhibitoren von Cystein-Proteasen, wie z.B. Calpain, zu erhalten. Weiterhin sind bei den vorliegenden Verbindungen der allgemeinen Formel I, die alle mindestens ein aliphatischen Amin-Rest tragen Salz-Bindungen mit Säuren möglich. Dies führt zu einer verbesserten Wasserlöslichkeit und damit zeigen die Verbindungen das gewünschte Profil für eine intravenöse Applikation, wie sie zum Beispiel bei der Schlaganfall-Therapie erforderlich ist.

Gegenstand der vorliegenden Erfindung sind heterozyklisch substituierte Amide der allgemeinen Formel I 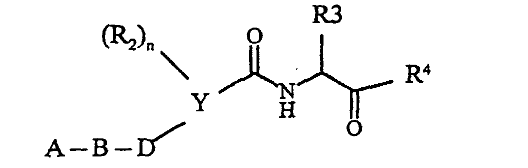 und ihre tautomeren und isomeren Formen, möglichen enantiomeren und diastereomeren Formen, sowie mögliche physiologisch verträgliche Salze, worin die Variablen folgende Bedeutung haben:
- A: - (CH₂)ₚ-R¹, wobei R¹ Pyrrolidin, Morpholin, Hexahydroazepin, Piperidin, -NR⁵R⁶ und 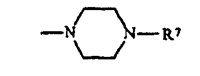 sein kann wobei die zyklischen Amine noch mit einem oder zwei Resten R¹⁵ substituiert sein können und R¹⁵ Wasserstoff, C₁-C₆-Alkyl, O-C₁-C₄-Alkyl und Phenyl bedeuten und R⁵, R⁶ und R⁷ unabhängig voneinander Wasserstoff,
C₁-C₄-Alkyl, Cyclohexyl, Cyclopentyl, CH₂Ph, Ph, CH₂CH₂Ph, wobei die Phenyl-Ringe noch mit R⁶ substituiert sein können und p 1 und 2 sein können und
- B: Phenyl, Pyridyl, Pyrazyl, Pyrimidyl und Pyridazyl bedeuten kann, wobei die Ringe noch mit zu bis 2 Resten R⁸ substituiert sein können, und
A und B zusammen auch 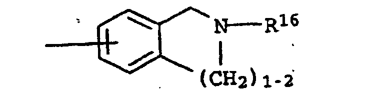 sein kann und R¹⁶ Wasserstoff, C₁-C₆-Alkyl und (CH₂)₁₋₄-Phenyl bedeutet, wobei der Phenyl-Ring noch mit maximal 2 Resten R⁶ substituiert sein kann, und
- D: -(CH₂)₀₋₂-O-(CH₂)₀₋₂, -(CH₂)ₘ -, CH=CH, -C≡C- sein kann und
- R²: Chlor, Brom, Fluor, C₁-C₆-Alkyl, NHCO-C₁-C₄-Alkyl, NHSO₂-C₁-C₄-Alkyl, NO₂, -O-C₁-C₄-Alkyl und NH₂ bedeutet, und
- R³: -C₁-C₆-Alkyl, verzweigt oder unverzweigt, und der noch einen SCH₃-Rest, einen Phenyl-Ring, Imidazolyl-Ring, Indolyl-Ring und Cyclopentyl-, Cycloheptyl-, Cyclohexyl-Ring tragen kann, der seinerseits mit maximal zwei Resten R⁸ substituiert ist, wobei R⁸ Wasserstoff, C₁-C₄-Alkyl, verzweigt oder unverzweigt, -O-C₁-C₄-Alkyl, OH, Cl, F, Br, J, CF₃, NO₂, NH₂, CN, COOH, COO-C₁-C₄-Alkyl, NHCO-C₁-C₄-Alkyl, -NHSO₂-C₁-C₄-Alkyl und -SO₂-C₁-C₄-Alkyl bedeutet; und
- Y: Phenyl und Pyridin bedeutet und
- R⁴: Wasserstoff, COOR⁹ und CO-Z bedeutet, worin Z NR¹⁰R¹¹, und 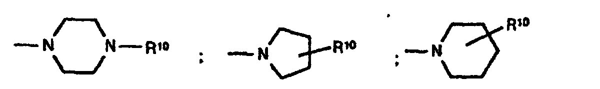 bedeutet,
- R⁹: Wasserstoff, C₁-C₆-Alkyl, geradlinig oder verzweigt, bedeutet und das mit einem Phenylring substituiert kann, der selbst noch mit einem oder zwei Resten R¹² substituiert sein kann, und
- R¹⁰: Wasserstoff, C₁-C₆-Alkyl, geradlinig oder verzweigt, bedeutet und das mit einem Phenylring substituiert kann, der selbst noch mit einem oder zwei Resten R¹² substituiert sein kann, und 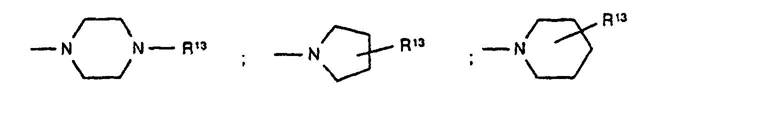 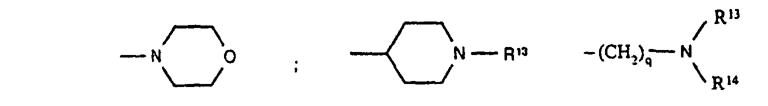
- R¹¹: Wasserstoff, C₁-C₆-Alkyl, verzweigt oder unverzweigt, das noch mit einem Phenylring, der noch einen Rest R⁹ tragen kann, und substituiert sein kann, bedeutet, und
- R¹²: Wasserstoff, C₁-C₄-Alkyl, verzweigt oder unverzweigt, -O-C₁-C₄-Alkyl, OH Cl, F, Br, J, CF₃, NO₂, NH₂, CN, COOH, COO-C₁-C₄-Alkyl, -NHCO-C₁-C₄-Alkyl, -NHCO-Phenyl, -NFiSO₂-C₁-C₄-Alkyl, -NHSO₂-Phenyl, -SO₂-C₁-C₄-Alkyl und -SO₂-Phenyl bedeuten kann
- R¹³: Wasserstoff, C₁-C₆-Alkyl, geradlinig oder verzweigt, bedeutet und das mit einem Phenylring substituiert kann, der selbst noch mit einem oder zwei Resten R¹² substituiert sein kann, und
- R¹⁴: Wasserstoff, C₁-C₆-Alkyl, geradlinig oder verzweigt, bedeutet und das mit einem Phenylring substituiert kann, der selbst noch mit einem oder zwei Resten R¹² substituiert sein kann, und
- n: eine Zahl 0, 1 oder 2 bedeutet,und
- m,q: unabhängig voneinander eine Zahl 0, 1, 2, 3 oder 4 bedeutet.

Bevorzugt werden die Verbindungen der allgemeinen Formel I, bei denen 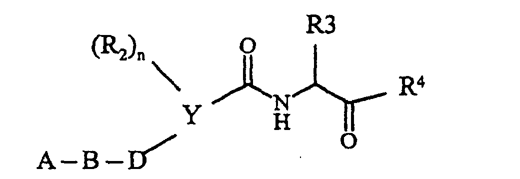
- A: -CH₂-R¹ , Wobei R¹ Pyrrolidin, Piperidin, -NR⁵R⁶ und 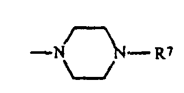 sein kann und R⁵, R⁶ und R⁷ unabhängig voneinander Wasserstoff und C₁-C₄-Alkyl sein können, und
- B: Phenyl
- D: -CH=CH-
- R²: Wasserstoff
- R³: Benzyl, CH₂CH₂CH₂CH₃, CH₂CH₂CH₂CH₂CH₃ und
- Y: Phenyl und Pyridin und
- R⁴: Wasserstoff und CO-NH₂ und
alle restlichen Variablen die gleiche Bedeutung wie im Anspruch 1 haben.

Die Verbindungen der Formel I können als Racemate, als enantiomerenreine Verbindungen oder als Diastereomere eingesetzt werden. Werden enantiomerenreine Verbindungen gewünscht, kann man diese beispielsweise dadurch erhalten, daß man mit einer geeigneten optisch aktiven Base oder Säure eine klassische Racematspaltung mit den Verbindungen der Formel I oder ihren Zwischenprodukten durchführt. Andererseits können die enantiomeren Verbindungen ebenfalls durch Einsatz von kommerziell erwerbbaren Verbindungen, zum Beispiel optisch aktive Aminosäuren wie Phenylalanin, Tryptophan und Tyrosin, hergestellt werden.

Gegenstand der Erfindung sind auch zu Verbindungen der Formel I mesomere oder tautomere Verbindungen, beispielsweise solche, bei denen die Aldehyd- oder Ketogruppe der Formel I als Enol-Tautomeres vorliegt.

Ein weiterer Gegenstand der Erfindung sind die physiologisch verträglichen Salze der Verbindungen I, die sich durch Umsatz von Verbindungen I mit einer geeigneten Säure oder Base erhalten lassen. Geeignete Säuren und Basen sind zum Beispiel in Fortschritte der Arzneimittelforschung, 1966, Birkhäuser Verlag, Bd. 10, S. 224-285, aufgelistet. Dazu zählen zum Beispiel Salzsäure, Citronensäure, Weinsäure, Milchsäure, Phosphorsäure, Methansulfonsäure, Essigsäure, Ameisensäure, Maleinsäure, Fumarsäure usw. bzw. Natriumhydroxid, Lithiumhydroxid,. Kaliumhydroxid und Tris.

Die Herstellung der erfindungsgemäßen Amide I, die eine Aldehyd-5 Gruppe tragen, kann auf verschiedenen Wegen erfolgen, die im Syntheseschema 1 skizziert wurde.

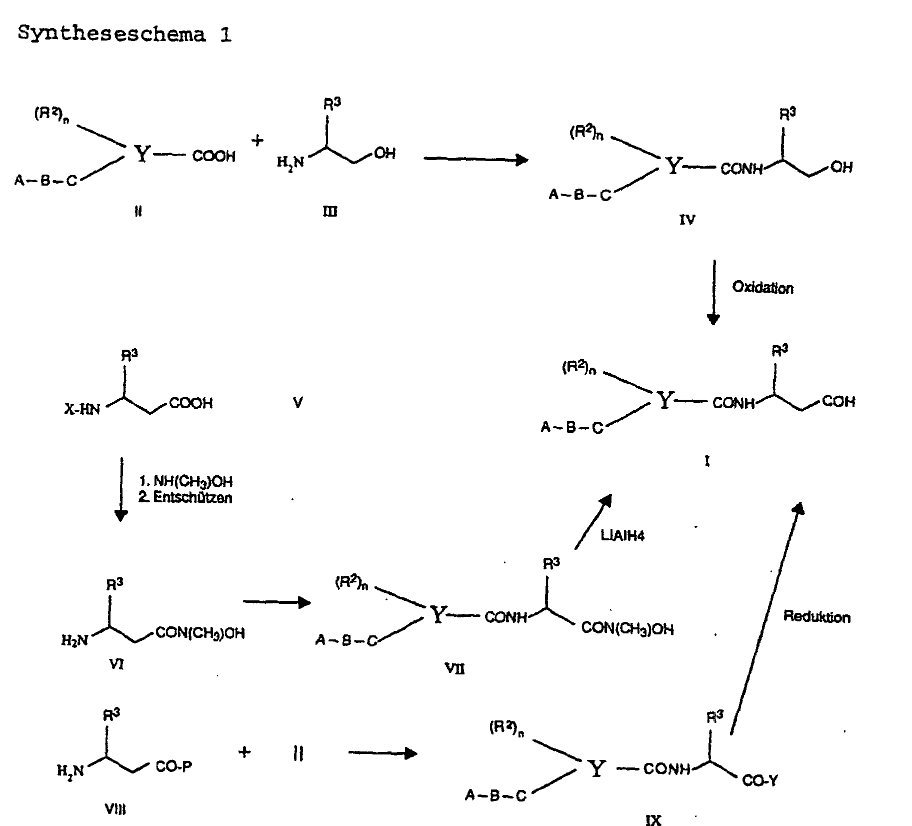

Heterocyclische Karbonsäuren II werden mit geeigneten Aminoalkoholen III zu den entsprechenden Amiden IV verknüpft. Dabei benutzt man übliche Peptid-Kupplungs-Methoden, die entweder im C.R. Larock, *Comprehensive Organic Transformations*, VCH Publisher, 1989, Seite 972f. oder im Houben-Weyl, Methoden der organischen Chemie, 4.Aufl., E5, Kap. V aufgeführt sind. Bevorzugt arbeitet man mit "aktivierten" Säurederivaten von II, wobei die Säuregruppe COOH in eine Gruppe COL überführt wird. L stellt eine Abgangsgruppe wie zum Beispiel Cl, Imidazol und N-Hydroxybenzotriazol dar. Diese aktivierte Säure wird anschließend mit Aminen zu den Amiden IV umgesetzt. Die Reaktion erfolgt in wasserfreien, inerten Lösungsmitteln wie Methylenchlorid, Tetrahydrofuran und Dimethylformamid bei Temperaturen von -20 bis +25°C.
Diese Alkohol-Derivate IV können zu den erfindungsgemäßen Aldehyd-Derivaten I oxidiert werden. Dafür kann man verschiedene übliche Oxidationsreaktionen (siehe C.R. Larock, *Comprenhensive Organic Transformations,* VCH Publisher, 1989, Seite 604 f.) wie zum Beispiel Swern- und Swern-analoge Oxidationen (T.T. Tidwell, *Synthesis,* 1990, 857-70), Natriumhypochlorid/TEMPO (S.L. Harbenson et al., siehe oben) oder Dess-Martin (*J. Org. Chem*. 1983, *48,* 4155) benutzen. Bevorzugt arbeitet man hier in inerten aprotischen Lösungsmitteln wie Dimethylformamid, Tetrahydrofuran oder Methylenchlorid mit Oxidationsmitteln wie DMSO / py x SO₃ oder DMSO / Oxalylchorid bei Temperaturen von -50 bis +25°C, je nach Methode (siehe obige Literatur). Alternativ kann man die Karbonsäure II mit Aminohydroxamsäure-Derivate VI zu Benzamiden VII umsetzten. Dabei bedient man sich der gleichen Reaktionsführung wie bei der Darstellung von IV. Die Hydroxam-Derivate VI sind aus den geschützten Aminosäuren V durch Umsatz mit einem Hydroxylamin erhältlich. Dabei benutzt auch hier ein bereits beschriebenes Amidherstellungsverfahren. Die Abspaltung der Schutzgruppe X, zum Beispiel Boc, erfolgt in üblicherweise, zum Beispiel mit Trifluoressigsäure. Die so erhaltenen Amid-hydroxamsäuren VII können durch Reduktion in die erfindungsgemäßen Aldehyde I umgewandelt werden. Dabei benutzt man zum Beispiel Lithiumaluminiumhydrid als Reduktionsmittel bei Temperaturen von -60 bis 0°C in inerten Lösungsmitteln wie Tetrahydrofuran oder Ether.
Analog zum letzten Verfahren kann man auch Karbonsäuren oder Säure-Derivate, wie Ester IX (P = COOR', COSR') herstellen, die ebenfalls durch Reduktion in die erfindungsgemäßen Aldehyde I überführt werden können. Diese Verfahren sind in R.C. Larock, *Comprehensive Organic Transformations*, VCH Publisher, 1989, Seite 619-26 aufgelistet.

Die Herstellung der erfindungsgemäßen heterozyklisch substituierten Amide I, eine Ketoamid- oder Ketoester-gruppe tragen, kann auf verschiedenen Wegen erfolgen, die in den Syntheseschemata 2 und 3 skizziert wurden.

Gegebenenfalls werden die Carbonsäureester IIa mit Säuren oder Basen wie Lithiumhydroxid, Natriumhydroxid oder Kaliumhydroxid in wäßrigen Medium oder in Gemischen aus Wasser und organischen Lösungsmitteln wie Alkohole oder Tetrahydrofuran bei Raumtemperatur oder erhöhten Temperaturen, wie 25-100°C, in die Säuren II überführt.

Diese Säuren II werden mit einem α-Aminosäure-Derivat verknüpft,wobei man übliche Bedingungen benutzt, die zum Beispiel im Houben-Weyl, Methoden der organischen Chemie, 4.Aufl., E5, Kap. V, und C.R. Larock, *Comprehensive Organic Transformations,* VCH Publisher, 1989, *Ch*. *9* aufgelistet sind.
Zum Beispiel werden die Carbonsäuren II in die "aktivierten" Säure-Derivate IIb (COOH → COL) überführt, wobei L eine Abgangsgruppe wie Cl, Imidazol und N-Hydroxybenzotriazol darstellt und anschließend durch Zugabe von einem Aminosäure-Derivat H₂N-CH(R³)-COOR in das Derivat XI überführt. Diese Reaktion erfolgt in wasserfreien, inerten Lösungsmitteln wie Methylenchlorid, Tetrahydrofuran und Dimethylformamid bei Temperaturen von -20 bis +25°C.

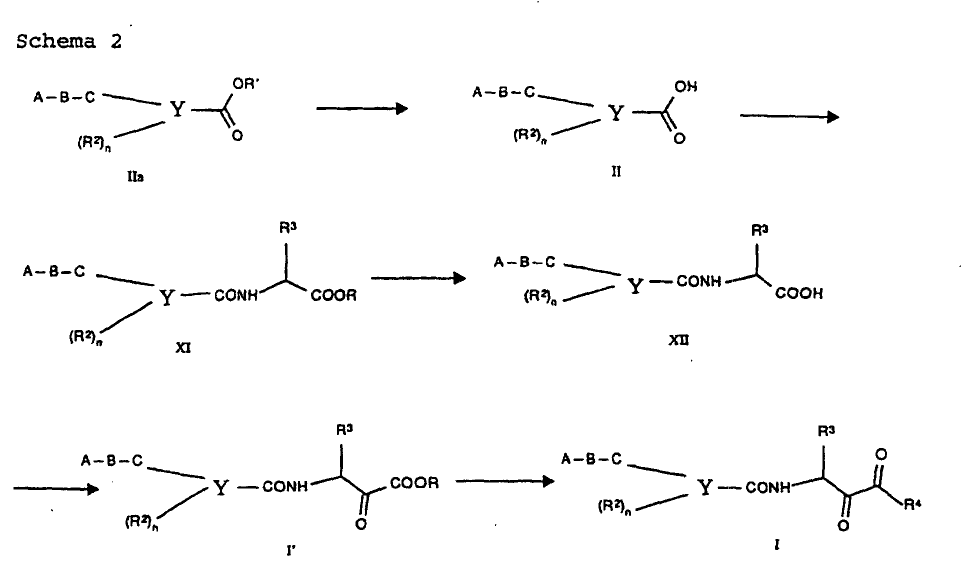

Die Derivate XI, die in der Regel Ester darstellen, werden analog der oben beschriebenen Hydrolyse in die Ketocarbonsäuren XII überführt. In einer Dakin-West analogen Reaktion werden die Ketoester I' hergestellt, wobei nach einer Methode von ZhaoZhao Li et al.. *J. Med. Chem*., 1993, *36,* 3472-80 gearbeitet wird. Dabei werden eine Karbonsäuren wie XII bei erhöhter Temperatur (50-100°C) in Lösungsmitteln, wie zum Beispiel Tetrahydrofuran, mit Oxalsäuremonoesterchlorid umgesetzt und anschließend das so erhaltene Produkt mit Basen wie Natriumethanolat in Ethanol bei Temperaturen von 25-80°C zum erfindungsgemäßen Ketoester I' umgesetzt. Die Ketoester I' können, wie oben beschrieben, zum Beispiel zu erfindungsgemäßen Ketocarbonsäuren hydrolysiert werden.

Die Umsetzung zu Ketobenzamiden I' erfolgt ebenfalls analog der Methode von ZhaoZhao Li et al. (s. oben). Die Ketogruppe in I' wird durch Zugabe von 1,2-Ethandithiol unter Lewissäure-Katalyse, wie zum Beispiel Bortrifluoridetherat, in inerten Lösungsmitteln, wie Methylenchlorid, bei Raumtemperatur geschützt, wobei ein Dithian anfällt. Diese Derivate werden mit Aminen R³-H in polaren Lösungsmitteln, wie Alkohole, bei Temperaturen von 0 bis 80°C umgesetzt, wobei die Ketoamide I (R⁴ = Z oder NR¹⁰R¹¹) anfallen.

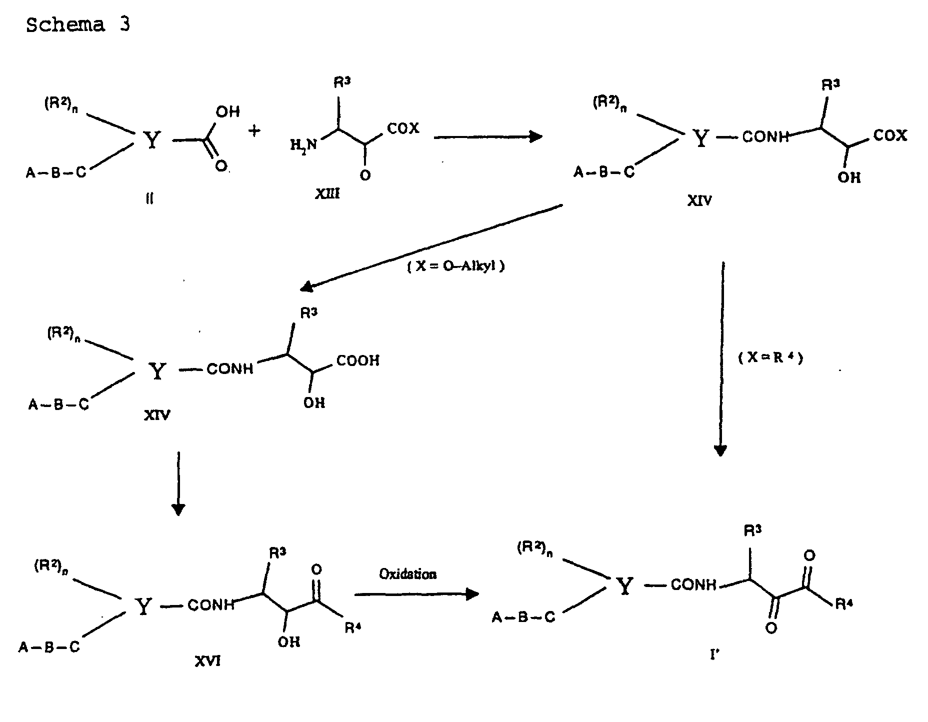

Eine alternative Methode ist im Schema 3 dargestellt. Die Ketocarbonsäuren II werden mit Aminohydroxykarbonsäure-Derivaten XIII (Herstellung von XIII siehe S.L. Harbenson et al., J. Med. Chem. 1994, 37, 2918-29 oder J.P. Burkhardt et al. Tetrahedron Lett. 1988, 29, 3433-3436) unter üblichen Peptid-Kupplungs-Methoden (siehe oben, Houben-Weyl) umgesetzt, wobei Amide XIV anfallen. Diese Alkohol-Derivate XIV können zu den erfindungsgemäßen Ketocarbonsäure-Derivaten I oxidiert werden. Dafür kann man verschiedene übliche Oxidationsreaktionen (siehe C.R. Larock, *Comprehensive Organic Transformations*, VCH Publisher, 1989, Seite 604f.) wie zum Beispiel Swern- und Swern-analoge Oxidationen, bevorzugt Dimethylsulfoxid/ Pyridin-Schwefeltrioxid-Komplex in Lösungsmitteln wie Methylenchlorid oder Tetrahydrofuran, gegebenenfalls unter Zusatz von Dimethylsulfoxid, bei Raumtemperatur oder Temperaturen von -50 bis 25°C, (T.T. Tidwell, *Synthesis* 1990, 857-70) oder Natriumhypochlorid/TEMPO (S.L. Harbenson et al., siehe oben), benutzen.

Wenn XIV α-Hydroxyester darstellen (X = O-Alkyl), können diese zu Karbonsäuren XV hydrolysiert werden, wobei analog zu den obigen Methoden gearbeitet wird, bevorzugt aber mit Lithiumhydroxid in Wasser/Tetrahydrofuran-Gemischen bei Raumtemperatur. Die Herstellung von anderen Estern oder Amiden XVI erfolgt durch Umsetzung mit Alkoholen oder Aminen unter bereits beschriebenen Kupplungsbedingungen. Das Alkohol-Derivat XVI kann erneut zu erfindungsgemäßen Ketocarbonsäure-Derivaten I oxidiert werden.

Die Herstellung der Carbonsäureester II sind teilweise bereits beschrieben worden oder erfolgt entsprechend üblicher chemischen Methoden.
Verbindungen, bei denen C eine Bindung darstellt, werden durch übliche aromatische Kupplung, zum Beispiel die Suzuki-Kupplung mit Borsäure-Derivaten und Halogenide unter Palladiumkatalyse oder Kupferkatalytische Kupplung von aromatischen Halogeniden, hergestellt. Die Alkyl-überbrückten Reste (C = -(CH₂)ₘ-) können durch Reduktion der analogen Ketone oder durch Alkylierung der Organolithium, z.B. ortho-Phenyloxazolidine, oder anderer Organometallverbindungen hergestellt werden (vgl. I.M. Dordor, et al., *J. Chem. Soc. Perkin Trans. I*, 1984, 1247-52).
Ether-überbrückte Derivate werden durch Alkylierung der entsprechenden Alkohole oder Phenole mit Halogeniden hergestellt. Alken- und Alkin- überbrückte Verbindungen werden zum Beispiel durch Heck-Reaktion aus aromatischen Halogeniden und entsprechenden Alkenen und Alkinen hergestellt (vgl. I. Sakamoto et al., *Chem*. *Pharm*. *Bull*., 1986, *34*, 2754-59).

Die in der vorliegenden Erfindung enthaltenen heterozyklisch substituierte Amide I stellen Inhibitoren von Cystein-Proteasen dar, insbesondere Cystein-Proteasen wie die Calpaine I und II und Cathepsine B bzw. L.

Die inhibitorische Wirkung der heterozyklisch substituierten Amide I wurde mit in der Literatur üblichen Enzymtests ermittelt, wobei als Wirkmaßstab eine Konzentration des Inhibitors ermittelt wurde, bei der 50% der Enzymaktivität gehemmt wird ( = IC₅₀).
Die Amide I wurden in dieser Weise auf Hemmwirkung von Calpain I, Calpain II und Cathepsin B gemessen.

### Cathepsin B-Test

Die Cathepsin B-Hemmung wurde analog einer Methode von S. Hasnain et al., *J. Biol. Chem*. 1993, *268*, 235-40 bestimmt.
Zu 88 µl Cathepsin B (Cathepsin B aus menschlicher Leber (Calbiochem), verdünnt auf 5 Units in 500 µM Puffer) werden 2 µl einer Inhibitor-Lösung, hergestellt aus Inhibitor und DMSO (Endkonzentrationen: 100 µM bis 0,01 µM). Dieser Ansatz wird für 60 Minuten bei Raumtemperatur (25°C) vorinkubiert und anschließend die Reaktion durch Zugabe von 10 µl 10 mM Z-Arg-Arg-pNA (in Puffer mit 10 % DMSO) gestartet. Die Reaktion wird 30 Minuten bei 405 nM im Mikrotiterplattenreader verfolgt. Aus den maximalen Steigungen werden anschließend die IC₅₀'s bestimmt.

### Calpain I und II Test

Die Testung der inhibitorischen Eigenschaften von Calpain-Inhibitoren erfolgt in Puffer mit 50 mM Tris-HCl, pH 7,5 ; 0,1 M NaCl; 1 mM Dithiotreithol; 0,11 mM Ca Cl₂, wobei das fluorogene Calpainsubstrats Suc-Leu-Tyr-AMC (25 mM gelöst in DMSO, Bachem/Schweiz) verwendet wird. Humanes µ-Calpain wird aus Erythrozyten isoliert und nach mehren chromatographischen Schritten (DEAE-Sepharose, Phenyl-Sepharose, Superdex 200 und Blue-Sepharose) erhält man Enzym mit einer Reinheit >95%, beurteilt nach SDS-PAGE, Western Blot Analyse und N-terminaler Sequenzierung. Die Fluoreszenz des Spaltproduktes 7-Amino-4-methylcoumarin (AMC) wird in einem Spex-Fluorolog Fluorimeter bei λex = 380 nm und λem = 460 nm verfolgt. In einem Meßbereich von 60 min. ist die Spaltung des Substrats linear und die autokatalytische Aktivität von Calpain gering, wenn die Versuche bei Temperaturen von 12° C durchgeführt werden. Die Inhibitoren und das Calpainsubstrat werden in den Versuchsansatz als DMSO-Lösungen gegeben, wobei DMSO in der Endkonzentration 2% nicht überschreiten soll.
In einem Versuchsansatz werden 10 µl Substrat (250 µM final) und anschließend 10 µl an µ-Calpain (2 µg/ml final, d.h.18 nM) in eine 1 ml Küvette gegeben, die Puffer enthält. Die Calpain-vermittelte Spaltung des Substrats wird für 15 bis 20 min. gemessen. Anschließend Zugabe von 10 µl Inhibitor (50 bis 100 µM Lösung in DMSO) und Messung der Inhibition der Spaltung für weitere 40 min.

Ki -Werte werden nach der klassischen Gleichung für reversible Hemmung bestimmt:
Ki = I / (v0/vi) - 1 ; wobei I= Inhibitorkonzentration, v0 = Anfangsgeschwindigkeit vor Zugabe des Inhibitors; vi = Reaktionsgeschwindigkeit im Gleichgewicht.

Die Geschwindigkeit wird errechnet aus v = Freisetzung AMC/Zeit d.h. Höhe/Zeit.

Calpain ist eine intrazelluläre Cysteinprotease. Calpain-Inhibitoren müssen die Zellmembran passieren, um den Abbau von intrazellulären Proteinen durch Calpain zu verhindern. Einige bekannte Calpain-Inhibitoren, wie zum Beispiel E 64 und Leupeptin, überwinden die Zellmembranen nur schlecht und zeigen dementsprechend, obwohl sie gute Calpain-Inhibitoren darstellen, nur schlechte Wirkung an Zellen. Ziel ist es, Verbindungen mit besser Membrangängigkeit zu finden. Als Nachweis der Membrangängigkeit von Calpain-Inhibitoren benutzen wir humane Plättchen.

### Calpain-vermittelter Abbau der Tyrosinkinase pp60src in Plättchen

Nach der Aktivierung von Plättchen wird die Tyrosinkinase pp60src durch Calpain gespalten. Dies wurde von Oda et al. in *J*. *Biol. Chem*., 1993, *268*, 12603-12608 eingehend untersucht. Hierbei wurde gezeigt, daß die Spaltung von pp60src durch Calpeptin, einen Inhibitor für Calpain, verhindert werden kann. In Anlehnung an diese Publikation wurde die zellulare Effektivität unserer Substanzen getestet. Frisches humanes, mit Zitrat versetztes Blut wurde 15 min. bei 200g zentrifugiert. Das Plättchen-reiche Plasma wurde gepoolt und mit Plättchenpuffer 1:1 verdünnt ( Plättchenpuffer: 68 mM NaCl, 2,7 mM KCl, 0,5 mM MgCl₂ x 6 H₂O, 0,24 mM NaH₂PO₄ x H₂O, 12 mM NaHCO₃, 5,6 mM Glukose, 1 mM EDTA, pH 7,4). Nach einem Zentrifugations- und Waschschritt mit Plättchenpuffer wurden die Plättchen auf 10⁷ Zellen/ml eingestellt. Die Isolierung der humanen Plättchen erfolgte bei RT.
Im Testansatz wurden isolierte Plättchen (2 x 10⁶) mit unterschiedlichen Konzentrationen an Inhibitoren (gelöst in DMSO) für 5 min. bei 37°C vorinkubiert. Anschließend erfolgte die Aktivierung der Plättchen mit 1µM Ionophor A23187 und 5 mM CaCl₂. Nach 5 min. Inkubation wurden die Plättchen kurz bei 13000 rpm zentrifugiert und das Pellet in SDS-Probenpuffer aufgenommen (SDS-Probenpuffer: 20 mM Tris-HCl, 5 mM EDTA, 5 mM EGTA, 1 mM DTT, 0,5 mM PMSF, 5 µg/ml Leupeptin, 10 µg/ml Pepstatin, 10 % Glycerin und 1% SDS). Die Proteine wurden in einem 12 %igen Gel aufgetrennt und pp60src und dessen 52-kDa und 47-kDa Spaltprodukte durch Western-Blotting identifiziert. Der verwendete polyklonale Kaninchen-Antikörper Anti-Cys-src (pp60c-rc) wurde von der Firma Biomol Feinchemikalien (Hamburg) erworben. Dieser primäre Antikörper wurde mit einem HRP-gekoppelten zweiten Antikörper aus der Ziege (Boehringer Mannheim, FRG) nachgewiesen. Die Durchführung des Western-Blotting erfolgte nach bekannten Methoden.
Die Quantifizierung der Spaltung von pp60src erfolgte densitometrisch, wobei als Kontrollen nicht-aktivierte (Kontrolle 1: keine Spaltung) und mit Ionophor- und Kalzium-behandelte Plättchen (Kontrolle 2: entspricht 100% Spaltung) verwendet wurden. Der ED₅₀ -Wert entspricht der Konzentration an Inhibitor bei der die Intensität der Farbreaktion um 50% reduziert wird.

### Glutamat induzierter Zelltod an corticalen Neuronen

Der Test wurde, wie bei Choi D.W., Maulucci-Gedde M.A. and Kriegstein A.R., "Glutamate neurotoxicity in cortical cell culture". *J*. *Neurosci*. 1989, 7, 357-368, durchgeführt.
Aus 15 Tage alten Mäuseembryos wurden die Cortexhälften präpariert und die Einzelzellen enzymatisch (Trypsin) gewonnen. Diese Zellen (Glia und corticale Neuronen) werden in 24 WellPlatten ausgesät. Nach drei Tagen (Laminin beschichteten Platten) oder sieben Tagen (Ornithin beschichteten Platten) wird mit FDU (5-Fluor-2-Desoxyuridine) die Mitosebehandlung durchgeführt. 15 Tage nach der Zellpräparation wird durch Zugabe von Glutamat (15 Minuten) der Zelltod ausgelöst. Nach der Glutamatentfernung werden die Calpaininhibitoren zugegeben. 24 Stunden später wird durch die Bestimmung der Lactatdehydrogenase (LDH) im Zellkulturüberstand die Zellschädigung ermittelt.

Man postuliert, daß Calpain auch eine Rolle im apoptotischen Zelltod spielt (M.K.T. Squier et al. *J*. *Cell*. *Physiol*. 1994, *159,* 229-237; T. Patel et al. *Faseb Journal* 1996, *590*, 587-597 ). Deshalb wurde in einem weiteren Modell in einer humanen Zellinie der Zelltod mit Kalzium in Gegenwart eines Kalziumionophors ausgelöst. Calpain-Inhibitoren müssen in die Zelle gelangen und dort Calpain hemmen, um den ausgelösten Zelltod zu verhindern.

### Kalzium-vermittelter Zelltod in NT2 Zellen

In der humanen Zellinie NT2 läßt sich durch Kalzium in Gegenwart des Ionophors A 23187 der Zelltod auslösen. 10⁵ Zellen/well wurden in Mikrotiterplatten 20 Stunden vor dem Versuch ausplattiert. Nach diesem Zeitraum wurden die Zellen mit verschiedenen Konzentrationen an Inhibitoren in Gegenwart von 2,5 µM Ionophor und 5 mM Kalzium inkubiert. Dem Reaktionsansatz wurden nach 5 Stunden 0,05 ml XTT (Cell Proliferation Kit II, Boehringer Mannheim) hinzugegeben. Die optische Dichte wird ungefähr 17 Stunden später, entsprechend den Angaben des Herstellers, in dem Easy Reader EAR 400 der Firma SLT bestimmt. Die optische Dichte, bei der die Hälfte der Zellen abgestorben sind, errechnet sich aus den beiden Kontrollen mit Zellen ohne Inhibitoren, die in Abwesenheit und Gegenwart von Ionophor inkubiert wurden.

Bei einer Reihe von neurologischen Krankheiten oder psychischen Störungen treten erhöhte Glutamat-Aktivitäten auf, die zu Zuständen von Übererregungen oder toxischen Effekten im zentralen Nervensystem (ZNS) führen. Glutamat vermittelt seine Effekte über verschiedene Rezeptoren. Zwei von diesen Rezeptoren werden nach den spezifischen Agonisten NMDA-Rezeptor und AMPA-Rezeptor klassifiziert. Antagonisten gegen diese Glutamat vermittelten Effekte können somit zur Behandlung dieser Krankheiten eingesetzt werden, insbesondere zur therapeutischen Anwendung gegen neurodegenerativen Krankheiten wie Chorea Huntington und Parkinsonsche Krankheit, neurotoxischen Störungen nach Hypoxie, Anoxie, Ischämie und nach Lesionen, wie sie nach Schlaganfall und Trauma auftreten, oder auch als Antiepileptika (vgl. *Arzneim. Forschung* 1990, *40,* 511-514; *TIPS,* 1990, *11*, 334-338; *Drugs of the Future* 1989, *14,* 1059-1071).

### Schutz gegen zerebrale Übererregung durch exzitatorische Aminosäuren (NMDA- bzw. AMPA-Antagonismus an der Maus)

Durch intrazerebrale Applikation von exzitatorischen Aminosäuren EAA (Excitatory Amino Acids) wird eine so massive Übererregung induziert, daß diese in kurzer Zeit zu Krämpfen und zum Tod der Tiere(Maus) führt. Durch systemische, z.B. intraperitoneale, Gabe von zentral-wirksamen Wirkstoffen (EAA-Antagonisten) lassen sich diese Symptome hemmen. Da die excessive Aktivierung von EAA-Rezeptoren des Zentralnervensystems in der Pathogenese verschiedener neurologischer Erkrankungen eine bedeutende Rolle spielt, kann aus dem nachgewiesenen EAA-Antagonismus in vivo auf eine mögliche therapeutische Verwendbarkeit der Substanzen gegen derartige ZNS-Erkrankungen geschlossen werden. Als Maß für die Wirksamkeit der Substanzen wurde ein ED₅₀-Wert bestimmt, bei dem 50% der Tiere durch eine festgelegte Dosis von entweder NMDA oder AMPA durch die vorangegangene ip.-Gabe der Meßsubstanz syptomfrei werden.

Die heterozyklisch substituierten Amide I stellen Inhibitoren von Cystein-Derivate wie Calpain I bzw. II und Cathepsin B bzw. L dar und können somit zur Bekämpfung von Krankheiten, die mit einer erhöhten Enzymaktivität der Calpain-Enzyme oder Cathepsin-Enzyme verbunden sind, dienen. Die vorliegenden Amide I können danach zur Behandlung von neurodegenerativen Krankheiten, die nach Ischämie, Schädigung durch Reperfusion nach Gefäßverschlüssen, Trauma, Subarachnoidal-Blutungen und Stroke auftreten, und von neurodegenerativen Krankheiten wie multipler Infarkt-Dementia, Alzheimer Krankheit, Huntington Krankheit und von Epilepsien und weiterhin zur Behandlung von Schädigungen des Herzens nach cardialen Ischämien, Schädigungen der Nieren nach renalen Ischämien, Skelettmuskelschädigungen, Muskeldystrophien, Schädigungen, die durch Proliferation der glatten Muskelzellen entstehen, coronaren Vasospasmen, cerebralen Vasospasmen, Katarakten der Augen, Restenosis der Blutbahnen nach Angioplastie dienen. Zudem können die Amide I bei der Chemotherapie von Tumoren und deren Metastasierung nützlich sein und zur Behandlung von Krankheiten, bei denen ein erhöhter Interleukin-1-Spiegel auftritt, wie bei Entzündungen und rheumatischen Erkrankungen, dienen.

Die erfindungsgemäßen Arzneimittelzubereitungen enthalten neben den üblichen Arzneimittelhilfsstoffen eine therapeutisch wirksame Menge der Verbindungen I.

Für die lokale äußere Anwendung, zum Beispiel in Puder, Salben oder Sprays, können die Wirkstoffe in den üblichen Konzentrationen enthalten sein. In der Regel sind die Wirkstoffe in einer Menge von 0,001 bis 1 Gew.-%, vorzugsweise 0,001 bis 0,1 Gew.-% enthalten.

Bei der inneren Anwendung werden die Präparationen in Einzeldosen verabreicht. In einer Einzeldosis werden pro kg Körpergewicht 0,1 bis 100 mg gegeben. Die Zubereitung können täglich in einer oder mehreren Dosierungen je nach Art und Schwere der Erkrankungen verabreicht werden.

Entsprechend der gewünschten Applikationsart enthalten die erfindungsgemäßen Arzneimittelzubereitungen neben dem Wirkstoff die üblichen Trägerstoffe und Verdünnungsmittel. Für die lokale äußere Anwendung können pharmazeutisch-technische Hilfsstoffe, wie Ethanol, Isopropanol, oxethyliertes Ricinusöl, oxethyliertes Hydriertes Ricinusöl, Polyacrylsäure, Polyethylenglykol, Polyethylenglykostearat, ethoxylierte Fettalkohole, Paraffinöl, Vaseline und wollfett, verwendet werden. Für die innere Anwendung eignen sich zum Beispiel Milchzucker, Propylenglykol, Ethanol, Stärke, Talk und Polyvinylpyrrolidon.

Ferner können Antioxidationsmittel wie Tocopherol und butyliertes Hydroxyanisol sowie butyliertes Hydroxytoluol, geschmacksverbessernde Zusatzstoffe, Stabilisierungs-, Emulgier- und Gleitmittel enthalten sein.

Die neben dem Wirkstoff in der Zubereitung enthaltenen Stoffe sowie die bei der Herstellung der pharmazeutischen Zubereitungen verwendeten Stoffe sind toxikologisch unbedenklich und mit dem jeweiligen Wirkstoff verträglich. Die Herstellung der Arzneimittelzubereitungen erfolgt in üblicher Weise, zum Beispiel durch Vermischung des Wirkstoffes mit anderen üblichen Trägerstoffen und Verdünnungsmitteln.

Die Arzneimittelzubereitungen können in verschiedenen Applikationsweisen verabreicht werden, zum Beispiel peroral, parenteral wie intravenös durch Infusion, subkutan, intraperitoneal und topisch. So sind Zubereitungsformen wie Tabletten, Emulsionen, Infusions- und Injektionslösungen, Pasten, Salben, Gele, Cremes, Lotionen, Puder und Sprays möglich.

### Beispiele

### Beispiel 1

(S)-2(E-2(4(N,N-Dimethylaminomethyl)phenyl)-ethen-1-yl)-N(3-phenyl-propan-1-al-2-yl)benzamid

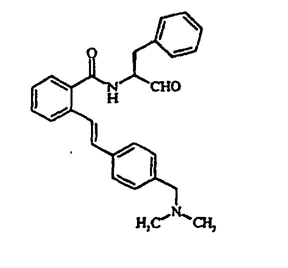

### a) 2 (E-2 (4(N,N-Dimethylaminomethyl)phenyl)-ethen-1-yl)-benzoesäureethylester

18,8 g (82 mmol) 2-Brombenzoesäureethylester, 17,2 g (107 mMol) 4-(N,N,-Dimethylaminomethyl)styrol, 20,7 g (205 mMol) Triethylamin, 0,36 g Palladium-II-acetat und 0,96 g Tri-(o-tolyl)phosphin wurden in 200 ml Dimethylformamid gegeben, mit 1 ml Wasser versetzt und für 3 h bei 140°C gerührt. Anschließend wurde das Reaktionsgemisch im Vakuum eingeengt und der anfallende Rückstand zwischen Essigester und Wasser verteilt. Die organische Phase wurde abgetrennt, mit Wasser gewaschen, getrocknet und im Vakuum eingeengt. Der Rückstand wurde noch aus Petrolether umkristallisiert. Man erhielt 16,1 g (63 %) des Produktes.

### b) 2 (E-2(4(N,N-Dimethylaminomethyl)phenyl)-ethen-1-yl)-benzoesäure

15,5 g (50 mMol) des Zwischenproduktes 1a wurden in 150 ml Ethanol gelöst und mit 50 ml 2M Natronlauge versetzt. Alles wurde 16 h bei Raumtemperatur gerührt. Anschließend wurde die Reaktionslösung mit 2M Salzsäure neutralisiert und das Ethanol im Vakuum entfernt. Der anfallende Niederschlag wurde abgesaugt und getrocknet. Man erhielt 13,6 g (97 %) des Produktes.

### c) (S)-2(E-2(4(N,N-Dimethylaminomethyl)phenyl)-ethen-1-yl)-N(3-phenyl-propan-1-ol-2-yl)-benzamid

1,97 g (7 mMol) des Zwischenproduktes 1b und 1,06 g (7 mMol) (S)-Phenylalaninol wurden in 25ml Methylenchlorid gegeben und mit 1,77 g (17,5 mMol) Triethylamin, und 0,95 g (7 mMol) 1-Hydroxybenzotriazol versetzt. Anschließend wurde bei 0°C 1,34 g (7 mMol) 1-Ethyl-3-(dimethylaminopropyl)carbodiimidhydrochlorid zugegeben und alles für 1h bei 0°C, dann 16 h bei Raumtemperatur gerührt. Der Reaktionsansatz wurde nacheinander mit 100 ml 5 %iger Zitronensäure und 100 ml Natriumhydrogenkarbonat-Lösung gewaschen und, nach dem Trocknen, im Vakuum eingeengt. Man erhielt 2,63 g (88 %) des Produktes.

### d) (S)-2(E-2(4(N,N-Dimethylaminomethyl)phenyl)-ethen-1-yl)-N(3-phenyl-propan-1-al-2-yl)-benzamid

2,40 g (5,6 mMol) der Zwischenverbindung 1c und 2,27 g (22,4 mMol) Triethylamin wurden in 25 ml trockenem Dimethylsulfoxid gelöst und mit 3,57 g (22,4 mMol) Pyridin-Schwefeltrioxid-Komplex versetzt. Alles wurde für 16 h bei Raumtemperatur gerührt. Anschließend wurde der Reaktiosnansatz auf wäßrige Natriumhydrogenkarbonat-Lösung gegeben und der Niederschlag abgesaugt. Die wäßrige Phase wurde noch mit Essigester extrahiert, der anschließend getrocknet und im Vakuum eingeengt wurde. Dieser Rückstand wurde mit dem ersten Niederschlag vereinigt.
Man erhielt 1,57 g (68 %) des Produktes.
¹H-NMR (D₆-DMSO): δ = 2,4 (6H), 2,8-3,1 (2H), 3,8 (1H), 7,0-7,7 (14H), 7,8 (1H), 8,8 (1H) und 9,75 (1H) ppm.

### Beispiel 2

### (S)-2(E-2(4(N,N-Dimethylaminomethyl)phenyl)-ethen-1-yl)-N(3-phenyl-propan-1-al-2-yl)-nicotinsäureamid

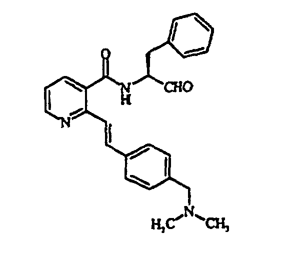

### a) 2(E-2(4(N,N-Dimethylaminomethyl)phenyl)-ethen-1-yl)-nicotinsäureethylester

6,7 g (39 mMol) 2-Chlornicotinsäureethylester, 8,2g (51 mMol) 4-(N,N,-Dimethylaminomethyl)styrol, 9,9 g (98 mMol) Triethylamin, 0,36 g Palladium-II-acetat und 0,96 Tri-(o-tolyl)phosphin wurden in 150 ml Dimethylformamid gegeben, mit 1 ml Wasser versetzt und für 13 h bei 140°C gerührt. Anschließend wurde das Reaktionsgemisch im Vakuum eingeengt und der anfallende Rückstand zwischen Essigester und Wasser verteilt. Die organische Phase wurde abgetrennt, mit Wasser gewaschen, getrocknet und im Vakuum eingeengt. Der Rückstand wurde noch aus Isopropanol nach Zugabe von einer äquivalenten Menge von Oxalsäure als Oxalat kristallisiert. Man erhielt 4,1 g (27 %) des Produktes als Monooxalat.

### b) 2(E-2(4(N,N-Dimethylaminomethyl)phenyl)-ethen-1-yl)-nicotinsäure

3,9 g (10 mMol) des Zwischenproduktes 2a wurden in 100 ml Ethanol/Tetrahydrofuran (1/1) gegeben und mit 25 ml 2M Natronlauge versetzt. Alles wurde 16h bei Raumtemperatur gerührt. Anschließend wurde die Reaktionslösung mit 2M Salzsäure neutralisiert und das Ethanol im Vakuum entfernt. Der anfallende Niederschlag wurde abgesaugt und getrocknet. Man erhielt 2,46 g (87 %) des Produktes.

### c) (S)-2(E-2(4(N,N-Dimethylaminomethyl)phenyl)-ethen-1-yl)-N(3-phenyl-propan-1-ol-2-yl)-nicotinsäuremid

2,03 g (7,2 mMol) des Zwischenproduktes 2b und 1,09 g (7,2 mMol) (*S*)-Phenylalaninol wurden in 25 ml Methylenchlorid gegeben und mit 1,82 g (18 mMol) Triethylamin, und 0,97 g (7,2 mMol) 1-Hydroxybenzotriazol versetzt. Anschließend wurde bei 0°C 1,38 g (7,2m Mol) 1-Ethyl-3-(dimethylaminopropyl)carbodiimidhydrochlorid zugegeben und alles für 1h bei 0°C, dann 16 h bei Raumtemperatur gerührt. Der Reaktionsansatz wurde nacheinander mit 100 ml 5 %iger Zitronensäure und 100 ml Natriumhydrogencarbonat-Lösung gewaschen und nach dem Trocknen im Vakuum eingeengt. Man erhielt 2,45 g (82 %) des Produktes.

### d) (S)-2(E-2(4(N,N-Dimethylaminomethyl)phenyl)-ethen-1-yl)-N(3-phenyl-propan-1-al-2-yl)-nicotinsäureamid

2,27 g (5,5 mMol) der Zwischenverbindung 2c und 2,21 g (21,85 mMol) Triethylamin wurden in 25 ml trockenem Dimethylsulfoxid gelöst und mit 3,48 g (21,85 mMol) Pyridin-Schwefeltrioxid-Komplex versetzt. Alles wurde für 16 h bei Raumtemperatur gerührt. Anschließend wurde der Reaktionsansatz auf wäßrige Natriumhydrogencarbonat-Lösung gegeben und der Niederschlag abgesaugt. Die wäßrige Phase wurde noch mit Essigester extrahiert, der anschließend getrocknet und im Vakuum eingeengt wurde. Dieser Rückstand wurde mit dem ersten Niederschlag vereinigt.
Man erhielt 1,4 g (61 %) des Produktes.

¹H-NMR (D₆-DMSO): δ = 2,15 (6H), 2,8 (1H), 3,3 (1H), 4,7 (1H), 6,9-7,8 (13H), 8,6 (1H), 9,0 (1H) und 9,7 (1H) ppm.

### Beispiel 3

N(1-Carbamoyl-1-oxo-3-phenyl-propan-2-yl)-2(E-2 (4 (morpholin-1-ylmethyl)phenyl)-ethen-1-yl)-benzamid

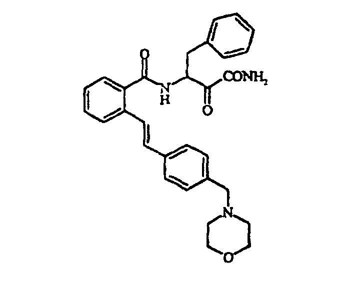

### a) N(4-Vinylphenyl)methylmorpholin

20 ml (0,14 Mol) 4-Vinylbenzylchlorid und 25 ml (0,28 Mol) Morpholin wurden für 3 h in 150 ml Methanol unter Rückfluß gekocht. Anschließend wurde alles im Vakuum eingeengt und der erhaltene Rückstand zwischen 1M Salzsäure und Wasser verteilt. Die salzsaure Phase wurde noch mit Ether gewaschen und danach mit 2M Natronlauge alkalisiert. Diese wäßrige Phase wurde mit Ether extrahiert. Diese organische Phase wurde getrocknet und im Vakuum eingeengt, wobei 24,6 g des Produktes anfielen.

### b) E-2(4(morpholin-1-ylmethyl)phenyl)-ethen-1-yl-benzoesäureethylester

14 g (68,9 mMol) der Zwischenverbindung 3a, 16,6 g (72,3 mMol) 2-Brombenzoesäure-ethylester, 24 ml (172 mMol) Triethylamin, 0,36 g Palladium-II-chlorid, 0,96 g tri-o-tolylphosphin und 1 ml Wasser wurden in 150ml Dimethylformamid für 2 h auf 100°C erwärmt. Anschließend wurde alles auf Wasser gegossen und die erhaltene Lösung mit Diethylether extrahiert. Die organische Phase wurde noch getrocknet und danach im Vakuum eingeengt, wonach 28 g des Produktes anfielen.

### c) E-2(4(Morpholin-1-ylmethyl)phenyl)-ethen-1-yl-benzoesäure x Hydrochlorid

28 g (80 mMol) der Zwischenverbindung 3b wurden 250 ml Ethanol gelöst und mit 9 g (159 mMol) Kaliumhydroxid, gelöst in 150 ml Wasser, versetzt. Alles wurde für 16 h bei Raumtemperatur gerührt. Anschließend wurde der Ansatz mit Salzsäure neutralisiert und mit Essigester extrahiert. Die organische Phase wurde getrocknet und im Vakuum eingeengt. Der Rückstand wurde in Ethanol gelöst und durch Zugabe von ethanolische Chlorwasserstoff-Lösung als Hydrochlorid gefällt, das anschließend abgesaugt wurde. Man erhielt 24,3 g des Produktes.

### d) N(1-Carbamoyl-1-hydroxy-3-phenyl-propan-2-yl)-2 (E-2 (4-(morpholin-1-ylmethyl)phenyl)-ethen-1-yl)-benzamid

1 g (2,8 mMol) der Zwischenverbindung 3c wurden analog der Vorschrift 2c mit 3-Amino-2-hydroxy-4-phenyl-buttersäureamid (J.P. Burkhardt et al., Tetrahedon Lett. 1988, 3433-3436) umgesetzt, wobei 0,97 g des Produktes erhalten wurden.

### e) N(1-Carbamoyl-1-oxo-3-phenyl-propan-2-yl)-2(E-2(4-(morpholin-1-ylmethyl)phenyl)-ethen-1-yl)-benzamid

0,9 g (1,8 mMol) der Zwischenverbindung 3d und 1 µl (7,2 mMol) Triethylamin wurden in 20 ml wasserfreiem Dimethylsulfoxid gelöst. Bei Raumtemperatur wurden anschließend 0,57 g (3,6 mMol) Pyridin-Schwefeltrioxid-Komplex, gelöst in 12 ml wasserfreiem Dimethylsulfoxid, zugetropft. Alles wurde noch für 30 Minuten gerührt. Danach wurde der Ansatz auf Wasser gegossen und mit wäßriger Natriumhydrogencarbonat-Lösung neutralisiert. Die wäßrige Phase wurde mir Essigester extrahiert. Die organische Phase wurde danach getrocknet und im Vakuum eingeengt. Der Rückstand wurde auf Aceton/Ether gefällt, wobei 0,51 g des Produktes ausfielen.

¹H-NMR (D₆-DMSO): δ = 2,3 (4H), 2,9 (1H), 3,25 (1H), 3,5 (2H); 3,6 (2H); 5,3 (1H), 7,0-7,6 (13H), 7,8 (2H), 8,1 (1H) und 8,9 (1H) ppm..

Analog den obigen Beispielen und Vorschriften wurden noch folgende Beispiele hergestellt:

### Beispiel 4

### N(1-Carbamoyl-1-oxo-3-phenyl-propan-2-yl)-2(E-2(4(1-pyrrolidinylmethyl)phenyl)-ethen-1-yl)-benzamid

¹H-NMR (CF₃COOH): δ = 2,15 (6H), 2,8 (1H), 3,3 (1H), 4,7 (1H), 6,9-7,8 (13H), 8,6 (1H), 9,0 (1H) und 9,7 (1H) ppm.

### Beispiel 5

### N(1-Carbamoyl-1-oxo-3-phenyl-propan-2-yl)-2(E-2(4-(N,N-diethylaminomethyl)-phenyl)-ethen-1-yl)-benzamid

¹H-NMR (D₆-DMSO): δ = 1,0 (6H); 2,5 (4H), 2,9 (1H), 3,25 (1H), 3,5 (2H); 5,4 (1H), 7,1-7,6 (13H), 7,8-7,9 (2H), 8,1 (1H) und 8,9 (1H) ppm.

### Beispiel 6

### 2(2E-(4(N,N-Benzylmethylaminomethyl)phenyl)-ethen-1-yl)-N-(1-carbamoyl-1-oxo-3-phenyl-propan-2-yl)-benzamid

¹H-NMR (D₆-DMSO): δ = 2,1 (3H), 2,9 (1H), 3,1-3,6 (5H), 5,3 (1H), 7,0-8,0 (16H), 8,1 (1H), und 8,9 (1H) ppm.

### Beispiel 7

### N(1-Carbamoyl-1-oxo-3-phenyl-propan-2-yl)-2(E-2(4(N,N-dimethylaminomethyl)-phenyl)-ethen-1-yl)-benzamid

1H-NMR (D₆-DMSO): δ = 2,5 (6H), 2,9 (1H), 3,3 (1H), 3,9 (2H); 5,4 (1H), 7,2-7,6 (15H), 8,9 (1H) und 8,9 (1H) ppm.

### Beispiel 8

### N(1-Carbamoyl-1-oxo-3-phenyl-propan-2-yl)-2 (E-2 (4 (N,N',-di-n-propyl-aminomethyl)-phenyl)-ethen-1-yl)-benzamid

¹H-NMR (D₆-DMSO): δ = 0,8 (6H); 1,5 (4H), 2,3 (2H); 2,9 (1H), 3,25 (1H), 3,5 (2H); 5,3 (1H), 7,1-7,5 (13H), 7,8 (2H), 8,1 (1H) und 8,9 (1H) ppm.

### Beispiel 9

### N(1-Carbamoyl-1-oxo-hexan-2-yl)-2(E-2(4(N,N-dimethylaminomethyl)-phenyl)-ethen-1-yl)-benzamid Hydrochlorid

¹H-NMR (D₆-DMSO): δ = 0,8 (3H); 1,2-1,9 (6H); 2,7 (6H), 4,2 (2H), 5,1 (1H), 7,1-8,0 (11H), 8,05 (1H) und 8,8 (1H) ppm.

### Beispiel 10

### N(1-Carbamoyl-1-oxo-3-phenyl-propan-2-yl)-2(E-2(4(4-methylpiperazin-1-yl-methyl)phenyl)-ethen-1-yl)-benzamid x Dihydrochlorid

¹H-NMR (D₆-DMSO): δ = 2,8-2,9 (3H), 3,1-3,8 (9H), 4,2 (2H), 5,3 (1H), 7,1-7,9 (17H), 8,1 (1H) und 8,9 (1H) ppm.

### Beispiel 11

### N(1-Carbamoyl-1-oxo-3-phenyl-propan-2-yl)-2(E-2(2(N,N-dimethylaminomethyl)-phenyl)-ethen-1-yl)-benzamid

¹H-NMR (D₆-DMSO): δ = 2,1 (6H), 2,9 (1H), 3,2 (1H), 3,5 (1H); 5.3 (1H), 7,0-8,0 (16H), 8,1 (1H) und 8,9 (1H) ppm.

### Beispiel 12

### N(1-Carbamoyl-1-oxo-3-phenyl-propan-2-yl)-2(E-2(4(N,N-dimethylaminomethyl)-phenyl)-ethen-1-yl)-nikotinsäureamid

¹H-NMR (D₅-DMSO): δ = 2,3 (6H), 2,85 (1H), 3,2 (1H), 3,7 (1H); 5,4 (1H), 7,2-7,6 (13H), 7,8 (1H), 8,6 (1H) und 9,15 (1H) ppm.

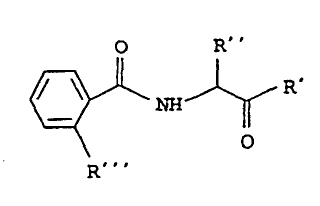

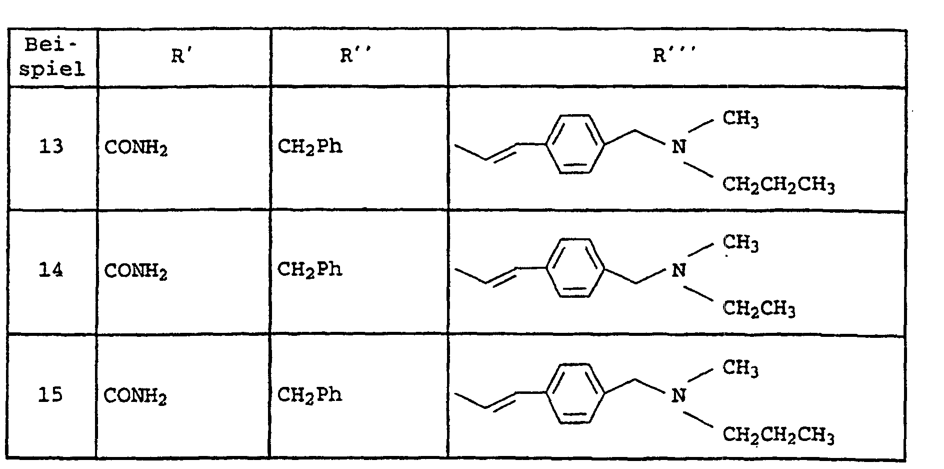

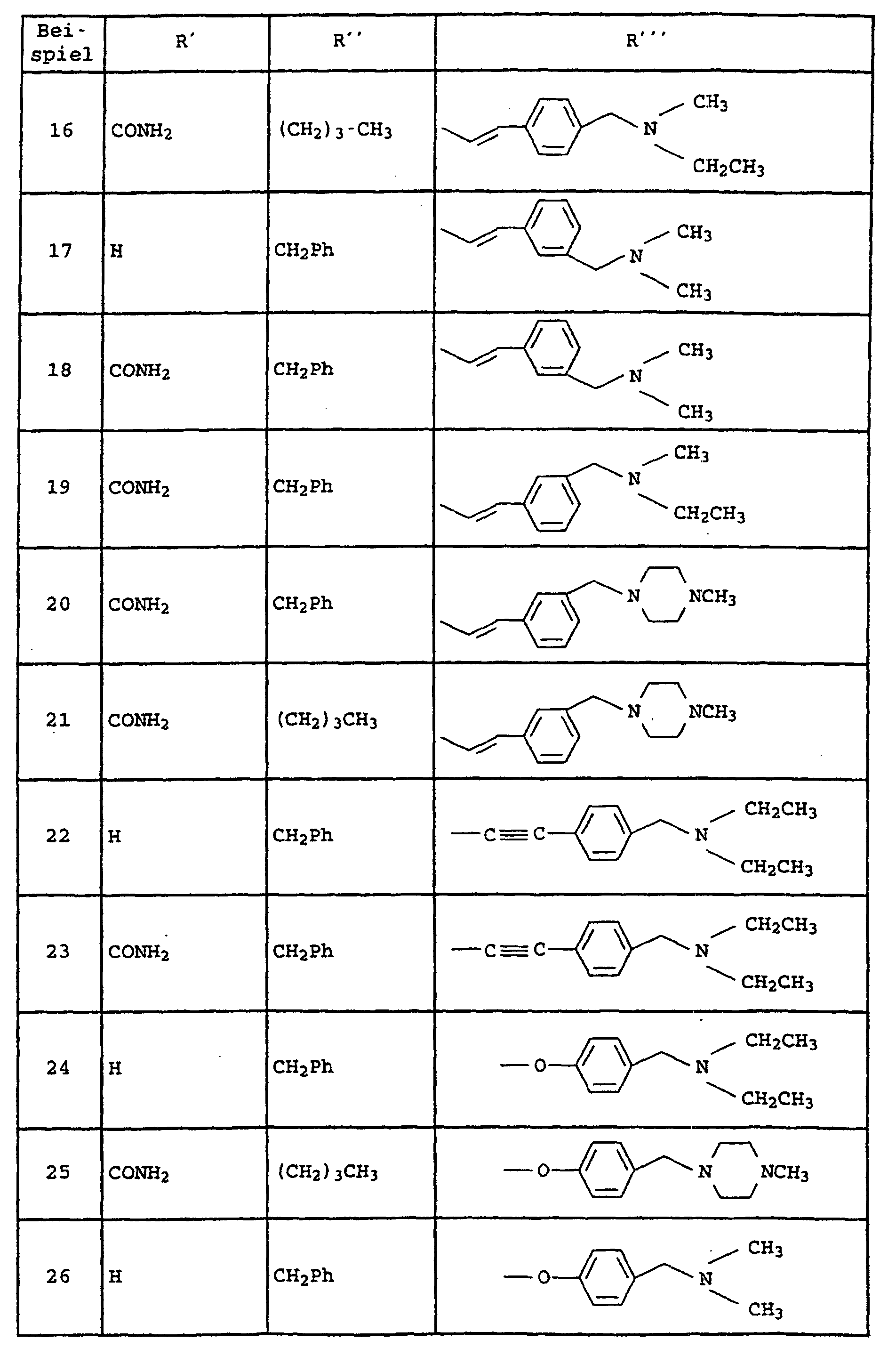

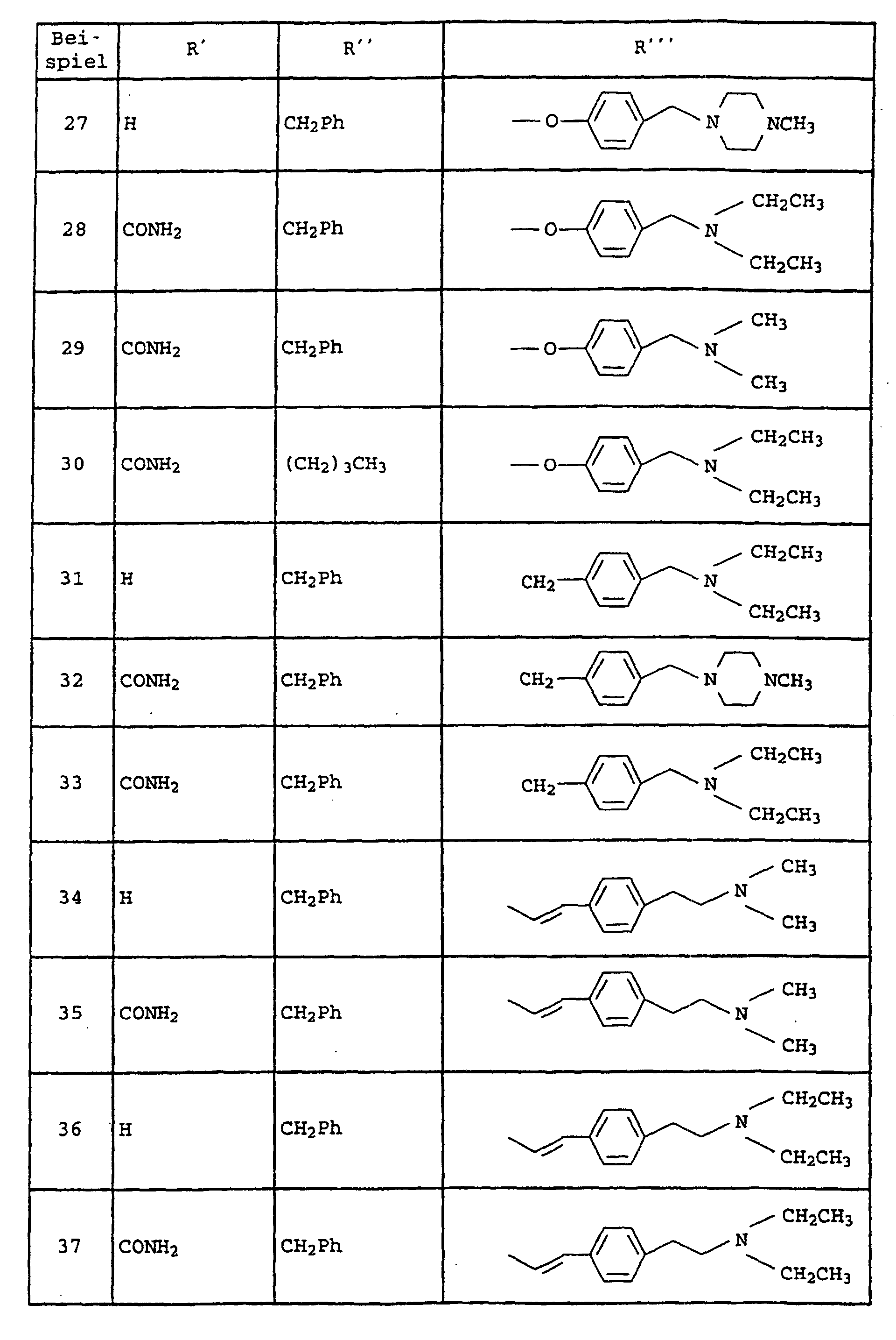

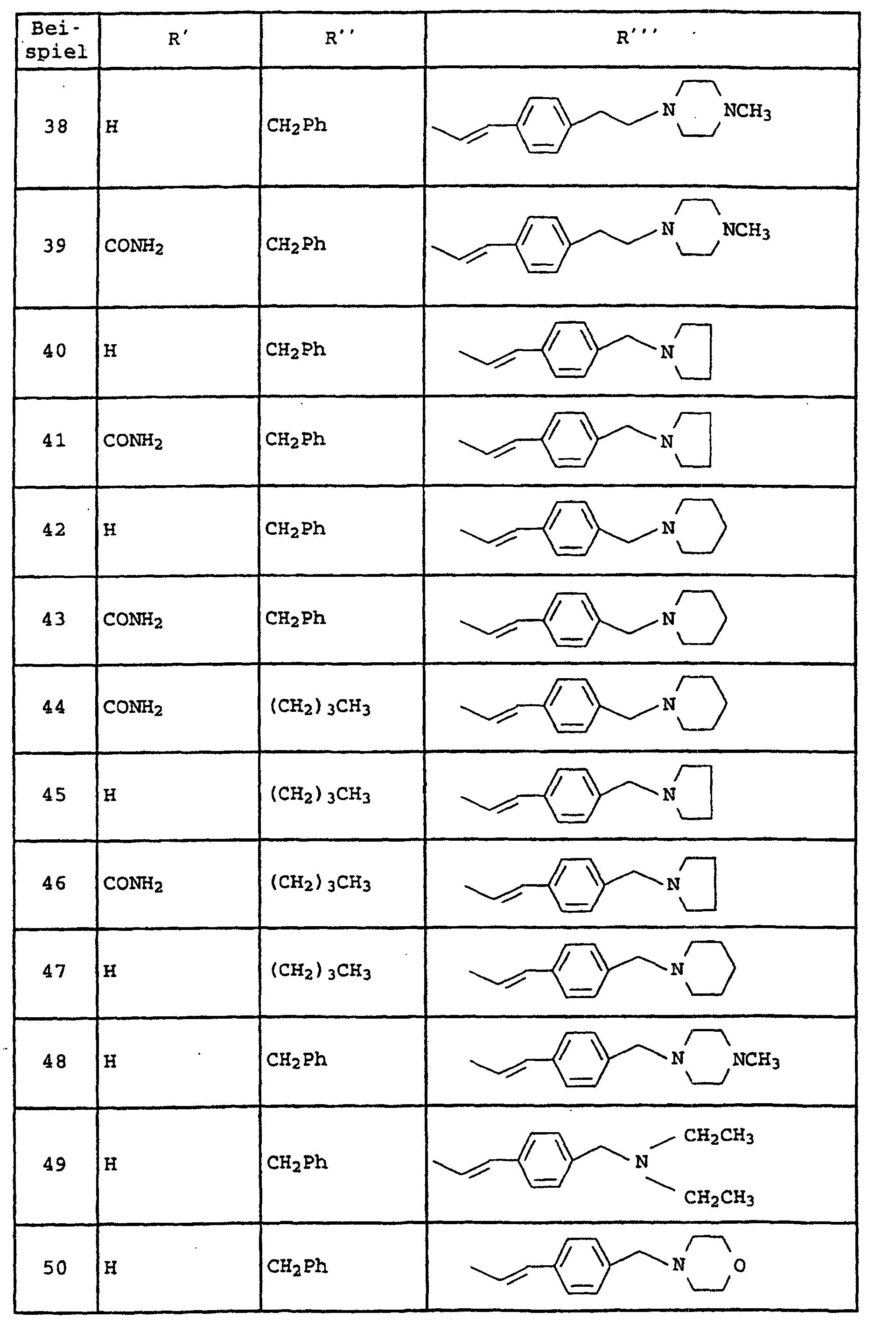

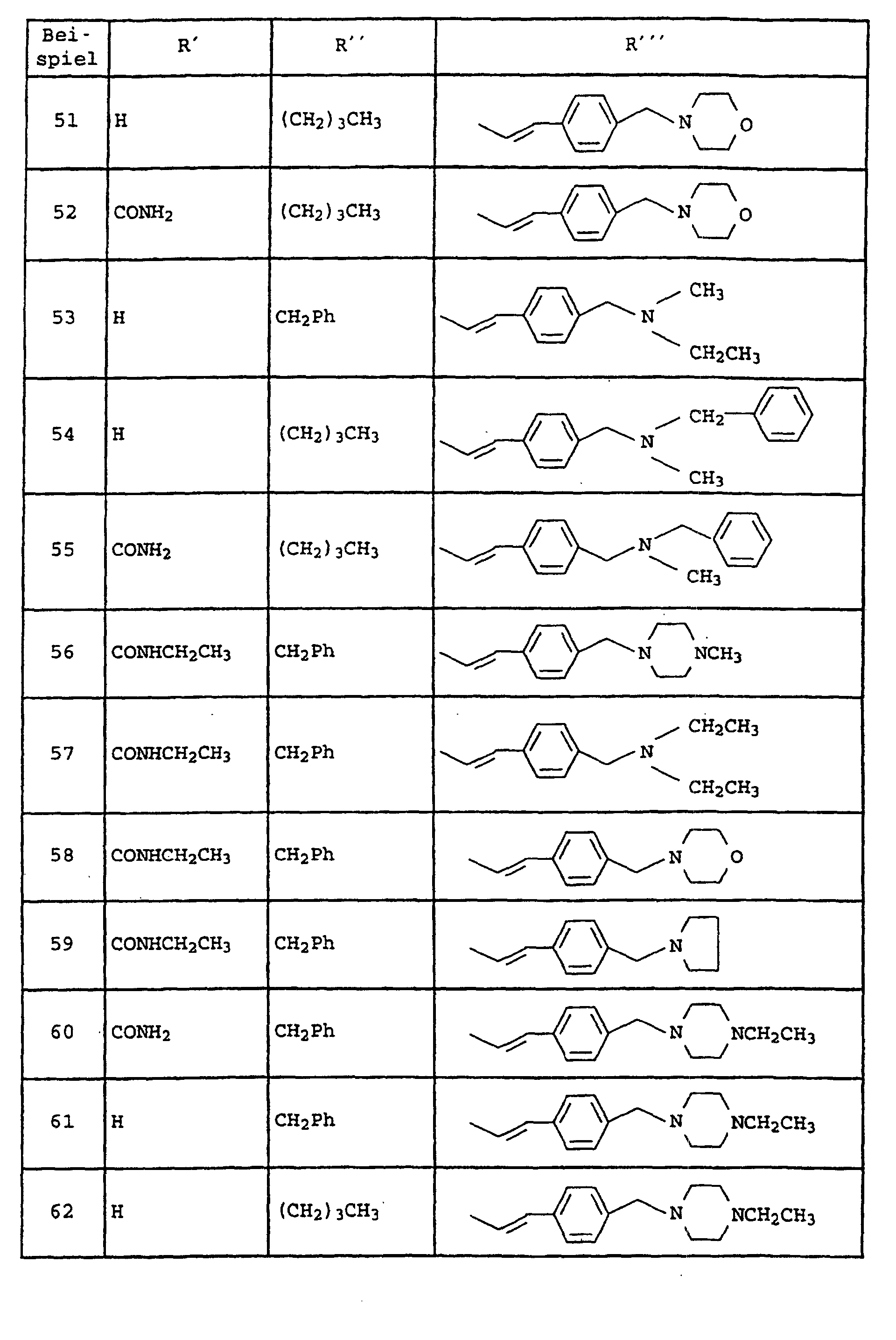

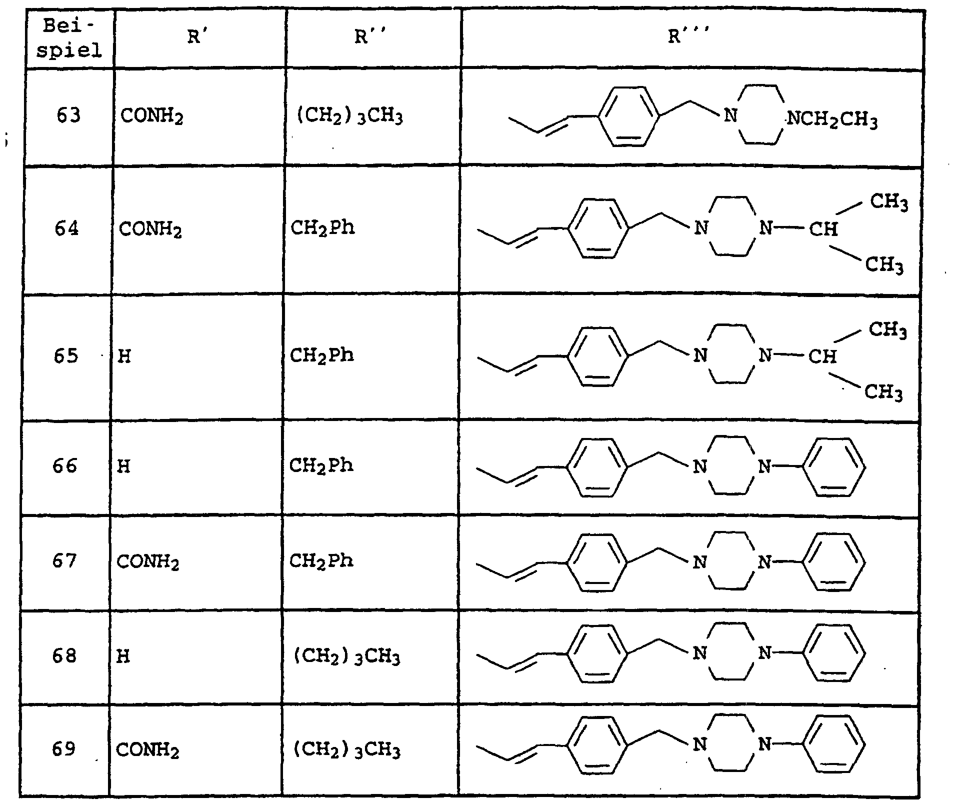

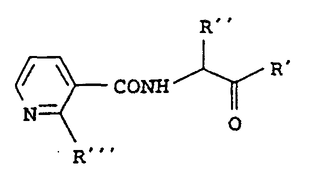

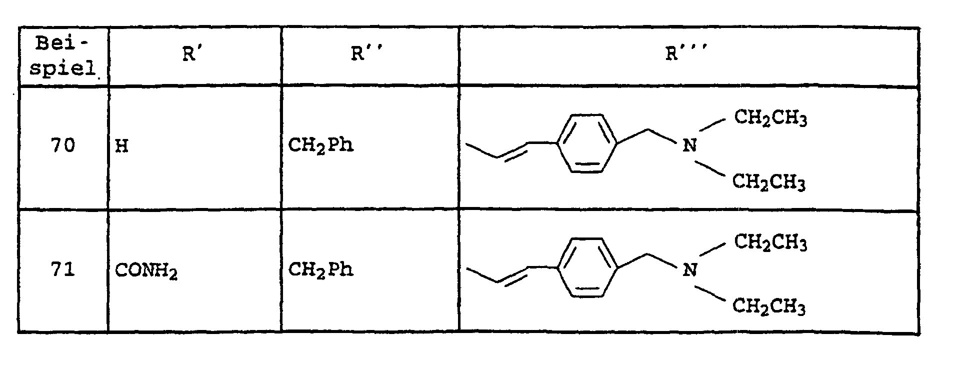

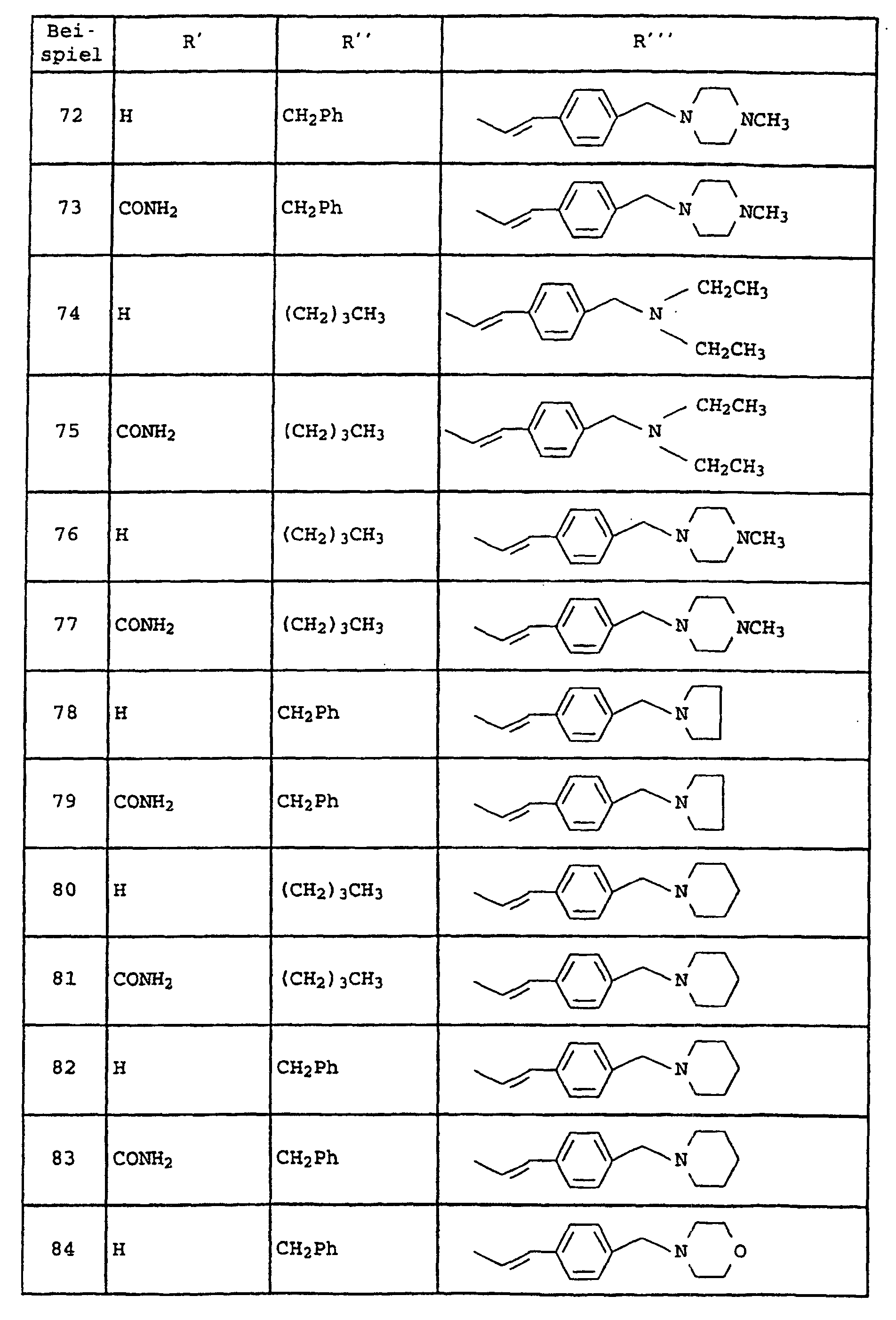

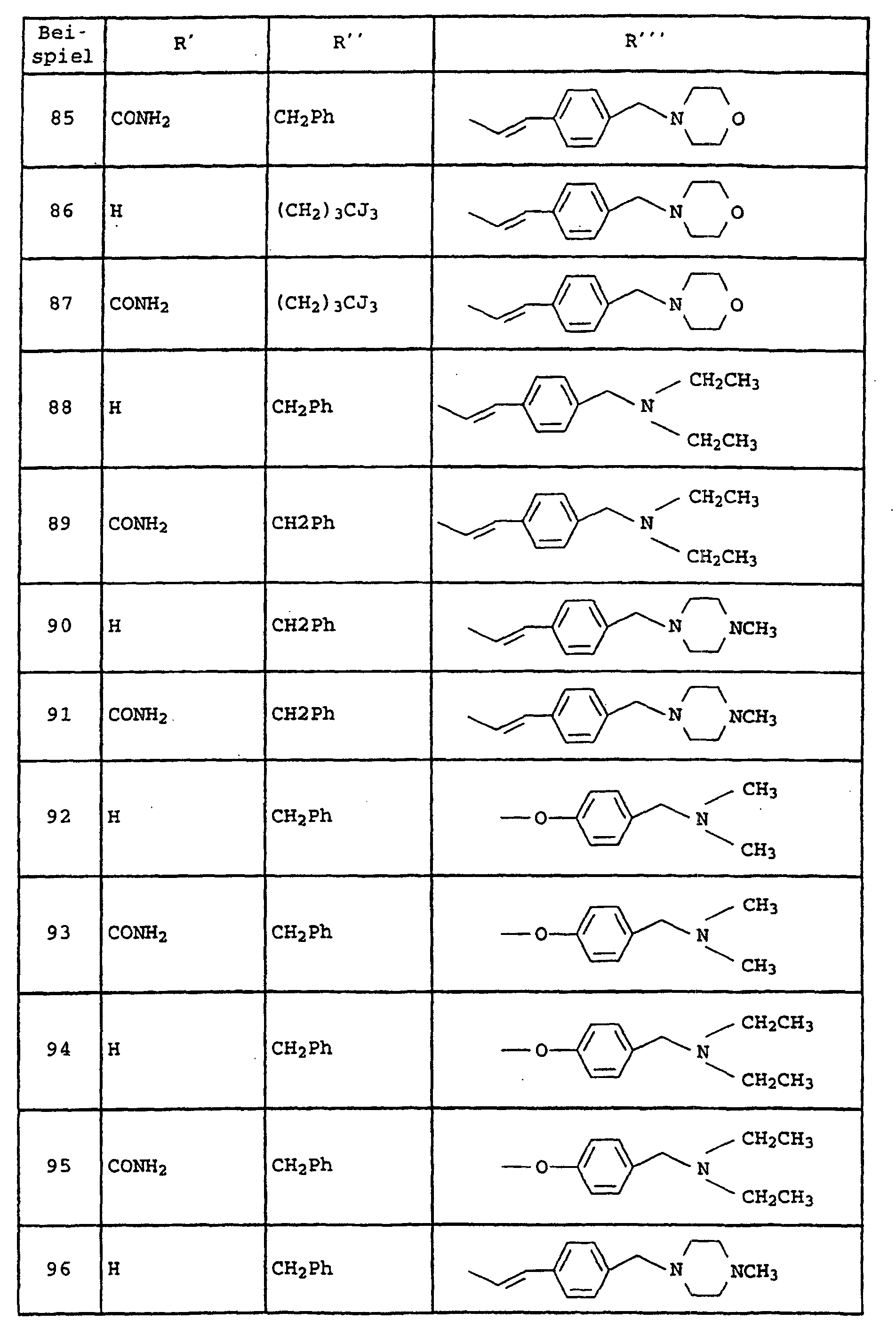

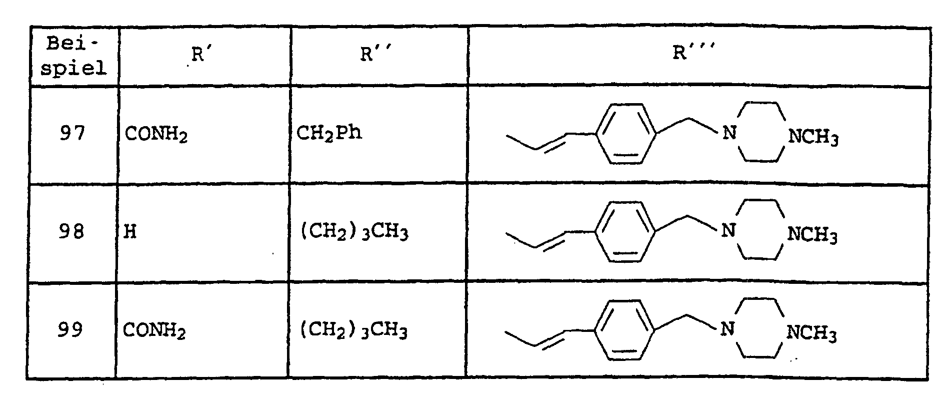

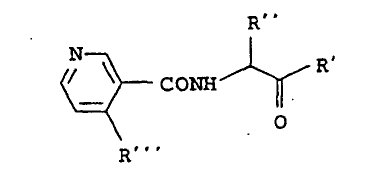

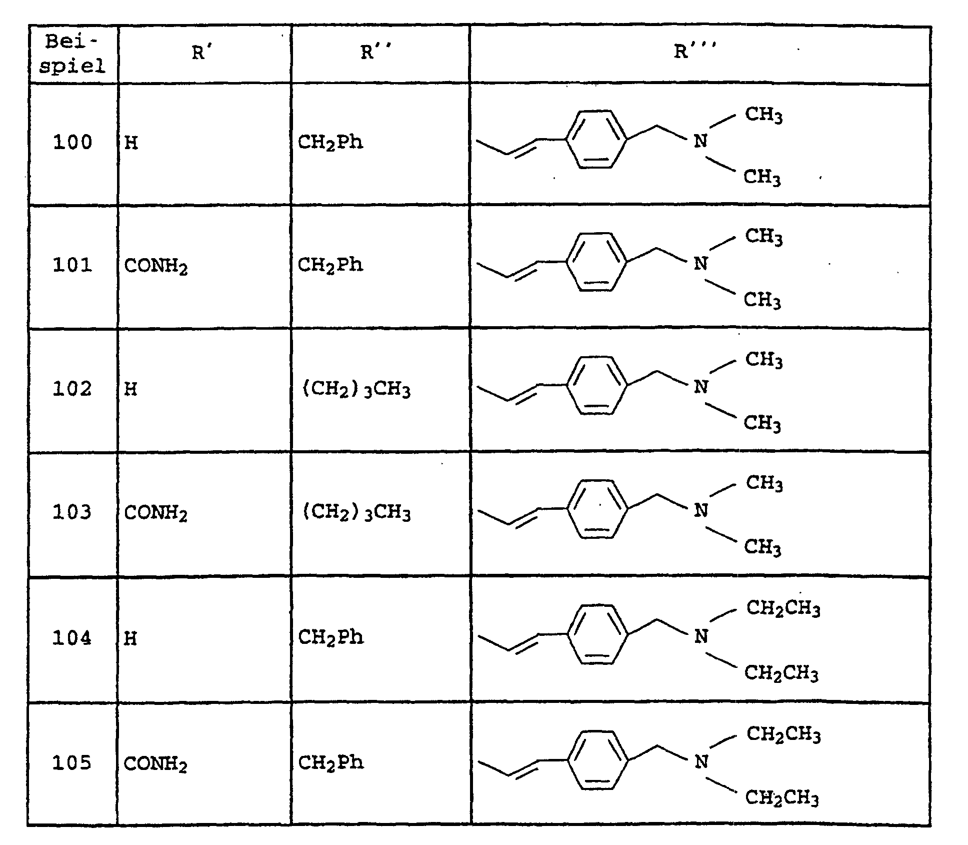

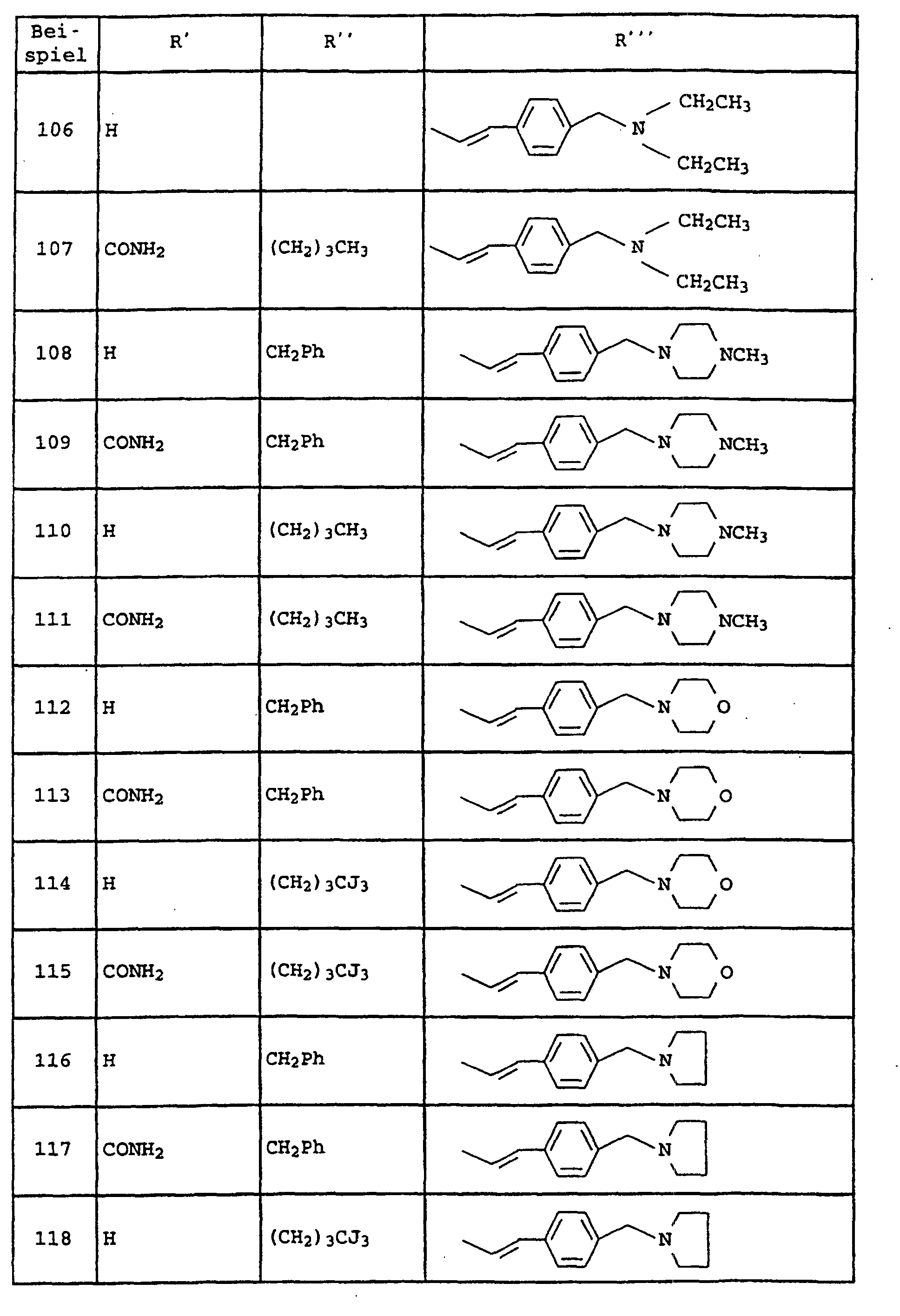

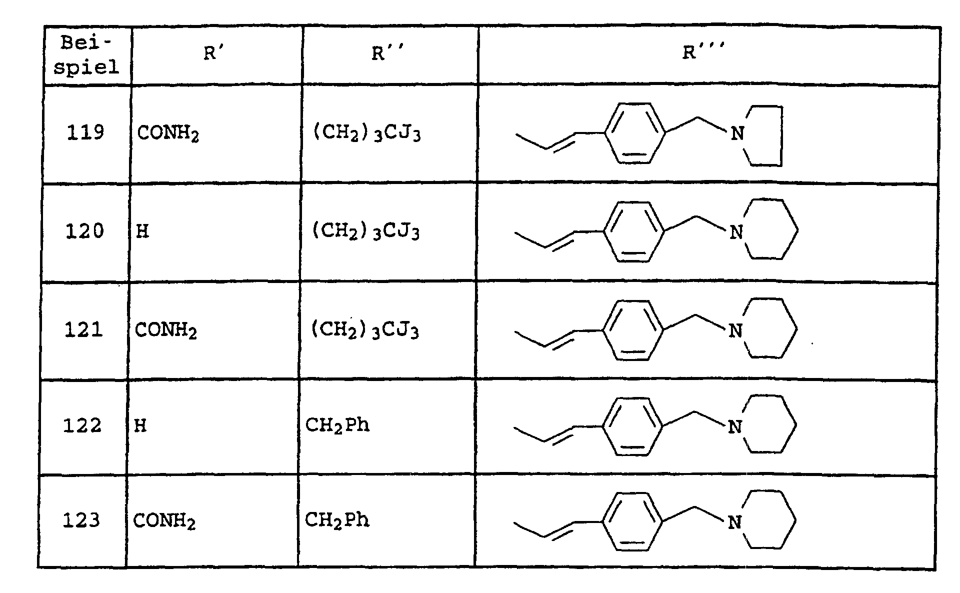

### Beispiel 44

### N(1-Carbamoyl-1-oxo-hexan-2-yl)-2(E-2(4(piperidin-1-yl-methyl)-phenyl)-ethen-1-yl)-benzamid

Ms: m/e = 462 (M⁺ + 1).

### Beispiel 60

### N(1-Carbamoyl-1-oxo-3-phenyl-propan-2-yl)-2(E-2(4(4-ethylpiperazin-1-ylmethyl)-phenyl)-ethen-1-yl)-benzamid

Ms: m/e = 524 (M⁺).

### Beispiel 66

### N(1-Carbamoyl-1-oxo-3-phenyl-propan-2-yl)-2(E-2(4(4-phenylpiperazin-1-ylmethyl)-phenyl)-ethen-1-yl)-benzamid

¹H-NMR (D₆-DMSO):δ = 2.4 (1H), 2.5 (4H), 2.9 (1H), 3.1 (4H), 3.3 (1H), 3.6 (2H), 5.4 (1H), 6.8 (1H), 6.9 (2H) und 7.1 - 8.0 (18H) ppm.

### Beispiel 71

### N(1-Carbamoyl-1-oxo-3-phenyl-propan-2-yl)-2(E-2(4(N,N-diethylaminomethyl)-phenyl)-ethen-1-yl)-nikotinsäureamid

¹H-NMR (D₆-DMSO):δ = 1.0 (6H), 2.85 (1H), 3.3 (1H), 3.6 (4H), 5.4 (1H), 7.2 - 8.0 (11H), 8.6 (1H) und 9.2 (1H) ppm.

### Beispiel 75

### N(1-Carbamoyl-1-oxo-hexan-2-yl)-2(E-2(4(N,N-diethylaminomethyl)-phenyl)-ethen-1-yl)-nikotinsäureamid

¹H-NMR (D₆-DNSO):δ = 1.0 (9H), 2.5 (4H), 3.5 (2H), 5.2 (1H), 7.3 - 8.2 (12H), 8.7 (1H) und 9.0 (1H) ppm.

### Beispiel 77

### N(1-Carbamoyl-1-oxo-hexan-2-yl)-2(E-2(4(4-methylpiperazin-1-ylmethyl)-phenyl)-ethen-1-yl)-benzamid

¹H-NMR (D₆-DMSO):δ = 0.9 - 1.9 (9H), 2.8 (4H), 5.2 (1H), 7.3 - 8.0 (12H), 8.1 (1H) und 8.8 (1H) ppm.

### Beispiel 79

### N(1-Carbamoyl-1-oxo-3-phenyl-propan-2-yl)-2 (E-2 (4 (pyrrolidin-1-ylmethyl)-phenyl)-ethen-1-yl)-nikotinsäureamid

¹-H-NMR (CF₃ COOD):δ = 2.1 - 2.4 (2H), 3.1 - 3.4 (3H), 3.6 - 3.9 (3H), 4.4 (2H), 5.2 (1H), 7.0 - 8.0 (16H) und 8.8 (1H) ppm.

### Beispiel 81

### N(1-Carbamoyl-1-oxo-hexan-2-yl)-2(E-2(4(piperidin-1-ylmethyl)-phenyl)-ethen-1-yl)-nikotinsäureamid

¹H-NMR (D₆-DMSO):δ = 0.9 - 1.9 (15H), 2.9 (2H), 3.2 (2H), 4.3 (2H), 5.2 (2H), 7.5 - 8.1 (11H), 8.8 (1H) und 9.0 (1H) ppm.

### Beispiel 83

### N(1-Carbamoyl-1-oxo-3-phenyl-propan-2-yl)-2(E-2(4(piperidin-1-ylmethyl)-phenyl)-ethen-1-yl)-nikotinsäureamid

¹H-NMR (CF₃ COOD):δ = 1.6 - 2.2 (6H); 3.0 - 3.2 (3H), 3.6 - 3.8 (2H), 4.3 (2H), 6.1 (1H), 7.0 - 8.0 (14H) und 8.8 (1H) ppm.

### Beispiel 85

### N(1-Carbamoyl-1-oxo-3-phenyl-propan-2-yl)-2(E-2(4(morpholin-1-ylmethyl)-phenyl)-ethen-1-yl)-nikotinsäureamid

¹H-NMR (D₆-DMSO):δ = 2.35 (2H), 2.8 (1H), 3.3 (1H), 3.5 (2H), 3.6 (2H), 5.4 (1H), 7.0 - 8.0 (14H), 8.1 (1H), 8.6 (1H) und 9.2 (1H) ppm.

### Beispiel 124

### N(1-Carbamoyl-1-oxo-3-phenyl-propan-2-yl)-2(E-2(4(N,N-diethylaminomethyl)-phenyl)-ethen-1-yl)-nikotinsäureamid x Dihydrochlorid

¹H-NMR (D₆-DMSO):δ = 1.1 (6H), 2.9 (1H), 3.1 (4H), 3.3 (1H), 4.3 (2H), 5.5 (1H), 7.2 - 8.0 (13H), 8.7 (2H), 9.3 (1H) und 10.8 (breit) ppm.

### Beispiel 125

### N(1-Carbamoyl-1-oxo-3-phenyl-propan-2-yl)-2(E-2(4(N,N-dimethylaminomethyl)-phenyl)-ethen-1-yl)-nikotinsäureamid x Dihydrochlorid

¹H-NMR (D₆-DMSO):δ = 2.7 (6H), 2.9 (1H), 3.2 (1H), 4.3 (2H), 5.5 (1H), 7.2 - 8.0 (16H) und 8.6 (1H) ppm.

### Beispiel 126

### N(Butan-1-al-2-yl)-2(E-2(4(N,N-dimethylaminomethyl)phenyl)-ethen-1-yl)-5-methoxybenzamid

¹H-NMR (CDCL₃):δ = 1.0 (3H) , 1.8 (1H), 2.1 (1H), 3.0 (6H) , 3.8 (3H), 4.6 (2H), 4.8 (1H), 6.4 (1H), 6.8 - 7.2 (3H), 7.3 - 7.8 (6H) und 9.7 (1H) ppm.

### Beispiel 127

### 2(E-2(4(N,N-Dimethylaminomethyl)phenyl)-ethen-1-yl)-5-methoxy-N(pentan-1-al-2-yl)-benzamid

### Beispiel 128

### N(3-Cyclohexyl-propan-1-al-2-yl)-2(E-2(4(piperidin-1-yl-methyl)phenyl)-ethen-1-yl)-benzamid

¹H-NMR (CDCL₃):δ = 1.0 (2H), 1.2 (3H), 1.5 (4H), 1.7 (8H), 1.8 (2H), 2.5 (3H), 3.6 (2H), 4.9 (1H), 6.2 (1H), 7.1 (1H), 7.3 (1H), 7.4 (2H), 7.5 (5H), 7.7 (1H) und 9.6 (1H) ppm.

### Beispiel 129

### N(4-Methylpentan-1-al-2-yl)-2(E-2(4(piperidin-1-yl-methyl)phenyl)-ethen-1-yl)-benzamid

¹H-NMR (CDCL₃):δ = 0.9 (3H), 1.0 (3H), 1.4 (3H), 1.6 (6H), 1.8 (2H), 2.4 (2H), 3.5 (2H), 4.8 (1H), 6.2 (1H), 7.0 (1H), 7.2 - 7.6 (8H), 7.7 (1H) und 9.7 (1H) ppm.

### Beispiel 130

### N(Pentaa-1-al-2-yl)-2(E-2(4(piperidin-1-yl-methyl)phenyl)-ethen-1-yl)-benzamid

¹H-NMR (CDCL₃):δ = 0.9 (3H) , 1.4 - 1.6 (10H), 2.4 (4H) , 3.4 (2H) , 4.8 (1H), 6.3 (1H), 7.0 (1H), 7.2 - 7.6 (7H), 7.7 (1H) und 9.7 (1H) ppm.

### Beispiel 131

### 2(E-2(4(N,N-Dimethylamino-methyl)phenyl)-ethen-1-yl)-N(3-phenyl-propan-1-al-2-yl)-5-methoxy-benzamid

¹H-NMR (CDCL₃):δ = 2.3 (6H), 3.3 (2H), 3.6 (2H), 3.8 (3H), 4.9 (1H), 6.5 (1H), 7.0 - 7.4 (13H), 8.5 (1H) und 9.7 (1H) ppm.

### Beispiel 132

### N(3- (3-Indolyl)-propan-1-al-2-yl)-2 (E-2 (4 (piperidin-1-yl-methyl)phenyl)-ethen-1-yl)-benzamid

¹H-NMR (CDCL₃):δ = 1.4 (2H), 1.6 (4H), 2.4 (4H), 3.4 (2H), 3.5 (2H), 5.1 (1H), 6.4 (1H), 6.9 (2H), 7.1 - 7.5 (11H), 7.6 (2H), 8.1 (1H) und 9.8 (1H) ppm.

### Beispiel 133

### N(3-(4-Imidazolyl)-propan-1-a1-2-yl)-2(E-2(4(piperidin-1-yl-methyl)phenyl)-ethen-1-yl)-benzamid

¹H-NMR (D₆-DMSO):δ = 1.4 (2H), 1.6 (4H) , 2.4 (4H), 3.4 (2H) , 4.1 (2H), 4.6 (1H), 7.1 (2H), 7.2 - 7.7 (11H), 7.8 (1H), 8.9 (1H) und 9.7 (1H) ppm.

### Beispiel 134

### N(3-Cyclohexyl-propan-1-al-2-yl)-2(E-2(4(morpholin-1-yl-methyl)phenyl)-ethen-1-yl)-benzamid

¹H-NMR (CDCL₃):δ = 0.8 - 1.7 (11H), 1.8 (2H), 2.8 (4H), 3.8 (6H), 4.9 (1H), 6.4 (1H), 7.0 (1H); 7.2 - 7.6 (8H), 7.7 (1H) und 9.6 (1H) ppm.

### Beispiel 135

### N(4-Methyl-pentan-1-al-2-yl)-2(E-2(4(morpholin-1-yl-methyl)phenyl)-ethen-1-yl)-benzamid

¹H-NMR (CDCL₃):δ = 1.0 (6H), 1.5 (2H), 2.1 (1H), 2.8 (4H), 3.7 - 3.9 (6H), 4.8 (1H), 6.3 (1H), 7.0 (1H), 7.2 - 7.8 (9H) und 9.7 (1H) ppm.

### Beispiel 136

### 2(E-2(4(Morpholin-1-yl-methyl)phenyl)-ethen-1-yl)-N(pentan-1-al-2-yl)-benzamid

¹H-NMR (CDCL₃):δ = 1.0 (3H), 1.5 (2H), 1.7 (2H), 2.4 (4H), 3.4 (2H), 3.7 (4H), 4.9 (1H), 6.3 (1H), 7.0 (1H), 7.2 - 7.6 (8H), 7.7 (1H) und 9.7 (1H) ppm.

### Beispiel 137

### N(1-Carbamoyl-1-oxo-3-phenyl-propan-2-yl)-2(E-2(4(pyrrolidon-1-yl-methyl)-phenyl)-ethen-1-yl)-benzamid x Methansulfonsäure

¹H-NMR (D₆-DMSO):δ = 1.8 - 2.1 (2H), 2.3 (3H), 2.6 - 2.9 (2H), 3.1 - 3.3 (2H), 4.25 (2H), 4.8 (1H), 7.0 - 8.0 (17H) und 9.8 (1H) ppm.

### Beispiel 138

### N(1-Carbamoyl-1-oxo-3-phenyl-propan-2-yl)-2(E-2(4(morpholin-1-ylmethyl)-phenyl)-ethen-1-yl)-benzamid x Methansulfonsäure

¹H-NMR (D₆-DMSO):δ = 2.3 (3H), 2.8 (1H), 3.2 (1H), 3.7 (2H) , 3.9 (2H), 4.2 (1H), 5.3 (1H), 7.0 - 7.7 (14H), 7.9 (2H), 8.1 (1H), 9.0 (1H) und 9.8 (breit) ppm.

### Beispiel 139

### N(3-Imidazolyl-propan-1-al-2-yl)-2(E-2(4(morpholin-1-yl-methyl)phenyl)-ethen-1-yl)-benzamid

¹H-NMR CDCL₃):δ = 2.4 - 2.8 (6H), 3.5 (2H), 3.7 (4H), 4.8 (1H), 6.6 - 7.6 (13H), 7.9 (1H) und 9.6 (1H) ppm.

### Beispiel 140

### N(3-Indolyl-propan-1-al-2-yl)-2(E-2(4(morpholin-1-yl-methyl)phenyl)-ethen-1-yl)-benzamid

¹H-NMR (D₆-DMSO):δ = 2.4 (6H), 3.4 (4H), 3.6 (4H), 4.7 (1H), 6.9 - 7.9 (16H), 8.1 (1H) und 9.7 (1H) ppm.

### Beispiel 141

### 2(E-2(4(N,N-Dimethylamino-methyl)phenyl)-ethen-1-yl)-N(3-indolyl-propan-1-al-2-yl)-benzamid

¹H-NMR (CDCL₃):δ = 2.3 (6H), 3.4 (4H), 5.1 (1H), 6.4 (1H), 6.9 (1H), 7.0 - 7.5 (13H), 7.6 (2H) und 9.6 (1H) ppm.

### Beispiel 142

### N(1-Carbamoyl-1-oxo-propan-2-yl)-2(E-2(4(N,N-dimethylamino-methyl)-phenyl)-ethen-1-yl)-benzamid Hydrochlorid

¹H-NMR (D₆-DMSO):δ = 1.3 (3H), 2.7 (6H), 4.3 (2H) , 5.1 (1H), 7.3 - 8.0 (11H), 8.1 (1H), 9.0 (1H) und 11.2 (breit) ppm.

### Beispiel 143

### N(1-Carbamoyl-2-oxo-propan-2-yl)-2(E-2(4(morpholin-1-yl-methyl)-phenyl)-ethen-1-yl)-nikotinsäureamid Dihydrochlorid

¹H-NMR (D₆-DMSO):δ = 1.4 (3H), 3.1 (2H), 3.2 (2H), 3.8 - 4.0 (4H), 4.4 (2H), 5.2 (1H), 7.5 - 8.2 (10H), 8.7 (1H), 9.2 (1H) und 11.6 (breit) ppm.

### Beispiel 144

### N(1-Carbamoyl-1-oxo-propan-2-yl)-2(E-2(4(morpholin-1-yl-methyl)-phenyl)-ethen-1-yl)-benzamid Hydrochlorid

¹H-NMR (D₆-DMSO):δ = 1.3 (3H), 3.1 (2H), 3.2 (2H), 3.8 (2H), 3.9 (2H), 4.3 (2H), 5.1 (1H), 7.3 - 8.0 (11H), 8.1 (1H), 8.9 (1H) und 11.4 (breit) ppm.

### Beispiel 145

### N(1-Carbamoyl-1-oxo-propan-2-yl)-2(E-2(4(4-methyl-piperazin-1-ylmethyl)-phenyl)-ethen-1-yl) -benzamid Dihydrochlorid

¹H-NMR (D₆-DMSO):δ = 1.35 (3H), 3.0 - 3.3 (4H), 3.8 - 4.0 (4H), 4.3 (2H), 5.1 (1H), 7.3 - 8.1 (12H), 8.9 (1H) und 11.5 (breit) ppm.

### Beispiel 146

### N(1-Carbamoyl-1-oxo-hexan-2-yl)-2(E-2(4(4-methyl-piperidin-1-ylmethyl)-phenyl)-ethen-1-yl)-benzamid

### Beispiel 147

### N(1-Carbamoyl-1-oxo-3-phenyl-propan-2-yl)-2(E-2(4(4-methyl-piperidin-1-yl-methyl)-phenyl)-ethen-1-yl)-benzamid

### Beispiel 148

### N(1-Carbamoyl-1-oxo-3-phenyl-propan-2-yl)-2(E-2(4(N-(n-propyl)-N(2-methyl-propan-1-yl)aminomethyl)-phenyl)-ethen-1-yl)-benzamid

¹H-NMR (CDCL₃):δ = 0.9 (9H), 1.4 (2H), 1.8 (1H), 2.2 (2H), 2.3 (2H), 3.2 - 3.6 (4H), 5.6 (1H), 5.9 (1H), 6.4 (1H) und 6.8 - 7.8 (16H) ppm.

### Beispiel 149

### N(1-Carbamoyl-1-oxo-hexan-2-yl)-2(E-2(4(N-(isopropyl)-N(n-propyl)aminomethyl)-phenyl)-ethen-1-yl)-benzamid

¹H-NMR (CDCL₃):δ = 0.8 (6H), 1.0 (6H), 1.2 - 1.4 (4H), 1.7 (1H), 2.0 (1H), 2.4 (3H), 3.0 (1H), 3.0 - 3.2 (1H), 3.6 (2H), 5.4 (1H), 5.8 (1H), 6.4 (1H), 6.8 (1H), 7.0 (1H), 7.2 - 7.4 (7H), 7.6 (1H) und 7.7 (1H) ppm.

### Beispiel 150

### N(1-Carbamoyl-1-oxo-hexan-2-yl)-2(E-2(4(N-(n-propyl)-N(2-methyl-propan-1-yl)aminomethyl)-phenyl)-ethen-1-yl)-benzamid

¹H-NMR (CDCl3) : δ 0.9 (12H), 1.2-1.5 (5H), 1.7 (2H), 2.1 (2H), 2.4 (4H), 3.5 (2H), 5.4 (1H), 5.8 (1H), 6.4 (1H), 6.8 (1H), 7.0 (1H) und 7.2-7.6 (9H) ppm.

### Beispiel 151

### N(1-Carbamoyl-1-oxo-3-phenyl-propan-2-yl)-2(E-2(4(N-(isopropyl)-N(n-propyl)aminomethyl)-phenyl)-ethen-1-yl)-benzamid

¹H-NMR (CDCl3) : δ 0.8 (3H), 1.2 (6H), 1.5 (2H), 2.4 (2H), 2.9-3.4 (3H), 3.6 (2H), 4.6 (1H), 5.8 (1H), 6.4 (1H) und 6.8-7.8 (16H) ppm.

### Beispiel 152

### N(1-Carbamoyl-1-oxo-3-phenyl-propan-2-yl)-2(E-2(4((3,5dimethylmorpholin-1-yl)methyl)-phenyl)-ethen-1-yl)-benzamid

¹H-NMR (D₆-DMSO) : δ 1.0 (6H), 1.7 (2H), 2.8-3.7 (8H), 5.5 (1H), 7.1-7.8 (15H), 8.1 (1H) und 9.0 (1H) ppm.

### Beispiel 153

### N(1-Carbamoyl-1-oxo-3-phenyl-propan-2-yl)-2(E-2(4(N,N-(dimethoxyeth-1-yl)aminomethyl)-phenyl)-ethen-1-yl)-benzamid Hydrochlorid

¹H-NMR (D₆-DMSO) : δ 3.3-3.8 (10H), 4.5 (2H), 5.5 (1H), 7.0-8.0 (17H) und 9.0 (1H) ppm.

### Beispiel 154

### 2(E-2-(4-(4-tert-Butyl-piperidin-1-yl-methyl)-phenyl)-ethen-1-yl)-N(1-carbamoyl-1-oxo-3-phenyl-propan-2-yl)-benzamid

¹H-NMR (CDCl3) : δ 0.9 (9H), 1.1 (1H), 1.6 (4H), 2.2 (2H), 3.2 (4H), 3.8 (2H), 5.6 (1H), 5.8 (1H), 5.9 (1H), 6.4 (1H), 6.9-7.6 (14H) und 7.7 (1H) ppm.

### Beispiel 155

### 2(E-2(4-(4-tert-Butyl-piperidin-1-yl-methyl)-phenyl)-N(1-carbamoyl-1-oxo-hexan-2-yl)ethen-1-yl)benzamid

¹H-NMR (CDCl3) : δ 0.9 (9H), 1.2-2.0 (9H), 2.5 (2H), 2.8 (2H), 3.2 (2H), 3.3 (1H), 3.5 (2H), 4.1 (2H), 5.4 (1H), 5.9 (1H), 6.4 (1H), 7.0 (1H), 7.2 (2H), 7.4-7.6 (7H) und 7.7 (1H) ppm.

### Beispiel 156

### 2 (E-2(4-N,N-n-Butyl-methylaminomethyl)-phenyl)-ethen-1-yl)-N(1-carbamoyl-1-oxo-hexan-2-yl)-benzamid

¹H-NMR (D₆-DMSO) : δ 0.7 (6H), 1.2 (6H), 1.4 (2H), 2.3 (6H), 2.5 (3H), 2.7 (4H), 4.0 (2H), 4.9 (1H), 5.8 (1H), 6.9-7.4 (8H), 7.7 (2H), 7.9 (2H) und 8.7 (1H) ppm.

### Beispiel 157

### 2(E-2(4-N,N-n-Butyl-methylaminomethyl)-phenyl)-ethen-1-yl)-N(1-carbamoyl-1-oxo-3-phenyl-propan-2-yl)-benzamid

### Beispiel 158

### N(1-Carbamoyl-1-oxo-3-phenyl-propan-2-yl)-(E-2(4-(N,N-n-propylmethylaminomethyl)-phenyl)-ethen-1-yl)-benzamid

### Beispiel 159

### N(1-Carbamoyl-1-oxo-3-phenyl-propan-2-yl)-2(E-2(4-(N,N-(2-methylbut-2-yl)-methylaminomethyl-phenyl)ethen-1-yl)-benzamid

### Beispiel 160

### N(1-Carbamoyl-1-oxo-hexan-2-yl)-2 (E-2(4-(N,N-(2-methylbut-2-yl)-methylaminomethyl)-phenyl)-ethen-1-yl)-benzamid

### Beispiel 161

### N(1-Carbamoyl-1-oxo-hexan-2-yl)-2(E-2(4-(N,N-n-propyl-methylaminomethyl)-phenyl)-ethen-1-yl)-benzamid

¹H-NMR (D₆-DMSO) : δ 0.8 (6H), 1.3 (4H), 1.7 (2H), 2.4-2.6 (5H), 2.8 (2H), 4.0-4.2 (2H), 5.1 (1H), 7.1-7.6 (9H), 7.8 (2H), 8.1 (1H) und 8.8 (1H) ppm.

### Beispiel 162

### 2(E-2(4-(N,N-n-Butyl-ethylaminomethyl)-phenyl)-ethen-1-yl) -N(1-carbamoyl-1-oxo-3-phenyl-propan-2-yl)-benzamid

### Beispiel 163

### N(1-Carbamoyl-1-oxo-3-phenyl-propan-2-yl)-2(E-2(4(hexahydroazepin-1-yl-methyl)-phenyl)-ethen-1-yl)-benzamid

### Beispiel 164

### N(1-Carbamoyl-1-oxo-n-hexan-2-yl)-2(E-2(4(hexahydroazepin-1-ylmethyl-phenyl)-ethen-1-yl)-benzamid

### Beispiel 165

### N(1-Carbamoyl-1-oxo-3-phenyl-propan-2-yl)-2(E-2(4(N,N-diethylaminomethyl)-phenyl)-ethen-1-yl)-benzamid x Methansulfonsäure

¹H-NMR (D₆-DMSO) : δ 1.2 (6H), 2.3 (3H), 2.9 (1H), 3.1 (4H), 3.2 (1H), 4.3 (2H), 5.4 (1H), 7.2-8.0 (15H), 8.2 (1H), 8.9 (1H) und 9.4 (1H) ppm.

### Beispiel 166

### 2(E-2(4-(N,N-n-Butyl-ethylaminomethyl)-phenyl)-ethen-1-yl)-N(1-carbamoyl-1-oxo-n-hexan-2-yl)-benazamid

¹H-NMR (D₆-DMSO) : δ 0.8 (6H), 1.2-1.5 (7H), 1.5-1.8 (4H), 2.6 (2H), 2.9 (2H), 3.0 (2H), 4.3 (2H), 5.2 (1H), 7.2-7.7 (9H), 7.8 (2H), 8.1 (1H) und 8.9 (1H) ppm.

### Beispiel 167

### N(1-Carbamoyl-1-oxo-3-phenyl-propan-2-yl)-2(E-2(4(N,N-diethylaminomethyl)-phenyl)-ethen-1-yl)-4-methyl-benzamid Hydrochlorid

MS : m/e = 469 (M+)

### Beispiel 168

### N(1-Carbamoyl-1-oxo-n-hexan-2-yl)-2(E-2(4(N-ethyl-N-isopropylaminomethyl)-phenyl)-ethen-1-yl)-benzamid

¹H-NMR (CDCl₃) : δ 0.5 (9H), 1.0 (3H), 1.3 (3H), 1.8 (2H), 2.1 (2H), 2.4 (4H), 3.5 (2H), 5.4 (1H), 5.7 (1H), 6.4 (1H), 6.8 (1H), 7.1 (1H), 7.2-7.6 (8H) und 7.7 (1H) ppm.

### Beispiel 169

### N(1-Carbamoyl-1-oxo-3-phenyl-propan-2-yl)-2(E-2(4(N-ethyl-N-isopropylaminomethyl)-phenyl)-ethen-1-yl)-benzamid

¹H-NMR (CDCl₃) : δ 0.9 (6H), 1.0 (3H), 1.8 (1H), 2.2 (2H), 2.4 (2H), 3.1 (2H), 3.6 (2H), 5.7 (1H), 6.4 (1H), 6.9-7.5 (16H) und 7.7 (1H) ppm.

### Beispiel 170

### N(1-Carbamoyl-1-oxo-n-hexan-2-yl)-2(E-2(4(N-cyclohexyl-N-methylaminomethyl)-phenyl)-ethen-1-yl)-benzamid

¹H-NMR (CDCl3) : d 0.8 (3H), 1.1-1.5 (9H), 1.6-2.1 (6H), 2.2 (3H), 2.5 (2H), 3.6 (2H), 5.4 (1H), 5.8 (1H), 6.4 (1H), 6.8 (1H), 7.0 (1H), 7.2-7.6 (8H) und 7.8 (1H) ppm.

### Beispiel 171

### N(1-Carbamoyl-1-oxo-n-hexan-2-yl)-2(E-2(4(N-methyl-piperazin-1-yl-methyl)-phenyl)-ethen-1-yl)-nikotinsäureamid Dihydrochlorid

¹H-NMR (D₆DMSO) : δ 0.9-1.9 (10H), 2.8 (2H), 4.4 (2H), 5.2 (1H), 7.4-8.2 (13H), 8.7 (1H) und 9.1 (1H) ppm.

### Beispiel 172

### N(1-Carbamoyl-1-oxo-3-phenyl-propan-2-yl)-2(E-2(4(methyl-piperazin-1-yl-methyl)-phenyl)-ethen-1-yl)-nikotinsäureamid Dihydrochlorid

MS : m/e = 511 (M+)

### Beispiel 173

### N(1-Carbamoyl-1-oxo-3-phenyl-propan-2-yl)-2(E-2(4(N-cyclohexyl-N-methylaminomethyl)-phenyl)-ethen-1-yl)-benzamid

¹H-NMR (CDCl₃) : δ 0.9 (2H), 1.1-1.4 (7H), 1.6 (1H), 1.8 (2H), 2.1 (2H), 2.4 (3H), 3.9 (2H), 5.5 (1H). 5.9 (1H), 6.4 (1H) und 6.8-7.8 (16H) ppm.

### Beispiel 174

### N(1-Carbamoyl-1-oxo-3-phenyl-propan-2-yl)-2 (E-2(4(morpholin-1-ylmethyl)-phenyl)-ethen-1-yl)-nikotinsäureamid Dihydrochlorid

¹H-NMR (D₆DMSO) : δ 2.8 (1H), 3.0-3.4 (5H), 3.8-4.0 (4H), 4.4 (2H), 5.5 (1H), 7.0-8.0 (13H), 8.2 (1H), 8.7 (1H), 8.7 (1H), 9.2 (1H) und 11.8 (breit) ppm.

### Beispiel 175

### N(1-Carbamoyl-1-oxo-3-phenyl-propan-2-yl)-2(E-2(4(N,N-dimethylamino-methyl)-phenyl-ethen-1-yl)-nikotinsäureamid Dihydrochlorid

¹H-NMR (D₆DMSO) : δ 1.3 (6H), 2.9 (1H), 3.0-3.2 (4H), 3.3 (1H), 4.3 (2H), 5.4 (1H), 7.2-8.0 (13H), 8.2 (1H), 8.7 (1H), 9.2 (1H) und 10.6 (breit) ppm.

### Beispiel 176

### N(1-Carbamoyl-1-oxo-3-phenyl-propan-2-yl)-2(E-2(4(1,2,5,6-tetrahydropyridin-1-yl-methyl)-phenyl)-ethen-1-yl)-benzamid

MS : m/e = 493 (M+)

### Beispiel 177

### N(1-Carbamoy)-1-oxo-3-phenyl-propan-2-yl)-3-chlor-2(E-2(4 (N,N-dimethylaminomethyl)-phenyl)-ethen-1-yl)-benzamid

### Beispiel 178

### N(1-Carbamoyl-1-oxo-3-phenyl-propan-2-yl)-2 (E-2(4(4-methyl-piperazin-1-yl-methyl)-phenyl)-ethen-1-yl)-benzamid x 2 Methansulfonsäure

¹H-NMR (D₆-DMSO): δ 2.4 (12H), 2.8-3.7 (11H), 4.5 (2H), 5.4 (1H), 7.2-8.0 (18H), 8.2 (1H) und 9.0 (1H) ppm.

### Beispiel 179

### N(1-Carbamoyl-1-oxo-n-butan-2-yl)-2 (E-2(4(morpholin-1-yl-methyl)-phenyl)-ethen-1-ylbenzamid Hydrochlorid

¹H-NMR (D₆-DMSO): δ 1.0 (3H), 1.6 (1H), 1.9 (1H), 3.0-3.4 (4H), 3.7-4.0 (4H), 4.3 (2H), 5.2 (1H), 7.2-8.2 (12H), 8.9 (1H) und 11.8 (breit) ppm.

### Beispiel 180

### N(1-Carbamoyl-3-methyl-1-oxo-n-butan-2-yl)-2(E-2(4(4-methyl-piperazin-1-yl-methyl) -phenyl)-ethen-1-yl-benzamid x 2 Methansulfonsäure

¹H-NMR (D₆-DMSO): δ 0.9-1.1 (6H), 2.3 (3H), 2.8 (3H), 3.0-3.8 (8H), 3.9 (2H), 5.1 (1H), 7.0-8.1 (12H) und 8.8 (1H) ppm.

### Beispiel 181

### N(1-Carbamoyl-3-methyl-1-oxo-n-butan-2-yl)-2(E-2(4(morpholin-1-yl-methyl)-phenyl)-ethen-1-yl)-benzamid x Methansulfonsäure

¹H-NMR (D₆-DMSO): δ 0.9-1.1 (6H), 2.3 (4H), 3.0-3.5 (4H), 3.6-4.0 (4H), 4.4 (2H), 5.2 (1H), 7.2-8.1 (12H), 8.8 (1H) und 9.8 (breit) ppm.

### Beispiel 182

### N(1-Carbamoyl-1-oxo-n-butan-2-yl)-2(E-2(4(4-methyl-piperazin-1-yl-methyl)-phenyl)-ethen-1-yl-benzamid Dihydrochlorid

¹H-NMR (D₆-DMSO): δ 1.0 (3H), 1.6 (1H), 1.9 (1H), 2.8 (3H), 3.3-3.8 (10H), 5.1 (1H), 7.3-8.1 (12H), und 8.8 (1H) ppm.

### Beispiel 183

### N(1-Carbamoyl-1-oxo-n-hexan-2(4(piperidin-1-yl-methyl)-phenyl)-benzamid

### Beispiel 184

### N(1-Carbamoyl-1-oxo-3-phenyl-propan-2-yl)-2(4(piperidin-1-yl-methyl)-phenyl)-benzamid

### Beispiel 185

### N(1-Carbamoyl-1-oxo-3-phenyl-propan-2-yl)-2(N-methyl-tetrahydroisochinolin-7-yl)oxy-nikotinsäureamid

Ms : m/e = 458 (M⁺)

### Beispiel 186

### N(1-Carbamoyl-1-oxo-3-phenyl-propan-2-yl)-2(N-methyl-tetrahydroisochinolin-7-yl)oxy-benzamid

Ms: m/e = 458 (M⁺)

### Beispiel 187

### N(3-Phenyl-propan-1-al-2-yl)-2(4(piperidin-1-yl-methyl)-benzyloxy)-nikotinsäureamid

### Beispiel 188

### 2(4(N,N-Dimethylaminomethyl)-benzyloxy)-N(3-phenyl-propan-1-al-2-yl)nikotinsäureamid

### Beispiel 189

### N(3-Phenyl-propan-1-al-2-yl)-2(4(4-methylpiperazin-1-yl-methyl)-benzyloxy)-nikotinsäureamid

### Beispiel 190

N(1-Carbamoyl-1-oxo-3-phenyl-propan-2-yl)-2(4(2(N,N,dimethylamino)-eth-1-yl))-phenyloxy-nikotinsäureamid Hxydrochlorid

## Patentansprüche

1. Amide der allgemeinen Formel I 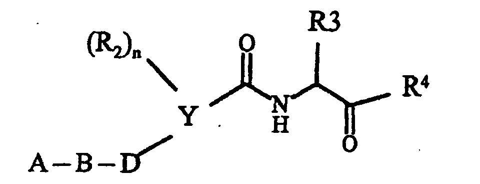 und ihre tautomeren Formen, möglichen enantiomeren und diastereomeren Formen, E- und Z-Formen, sowie mögliche physiologisch verträgliche Salze, worin die Variablen folgende Bedeutung haben:
A -(CH₂)ₚ-R¹, wobei R¹ Pyrrolidin, Morpholin, Hexahydroazepin, Piperidin, -NR⁵R⁶ und 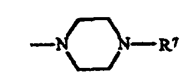 sein kann wobei die zyklischen Amine noch mit einem oder zwei Resten R¹⁵ substituiert sein können und R¹⁵ Wasserstoff, C₁-C₆-Alkyl, O-C₁-C₄-Alkyl und Phenyl bedeuten und R⁵, R⁶ und R⁷ unabhängig voneinander Wasserstoff, C₁-C₄-Alkyl, Cyclohexyl, Cyclopentyl, CH₂Ph, Ph, CH₂CH₂Ph, wobei die Phenyl-Ringe noch mit R⁶ substituiert sein können und p 1 und 2 sein können und
B Phenyl, Pyridyl, Pyrazyl, Pyrimidyl und Pyridazyl bedeuten kann, wobei die Ringe noch mit zu bis 2 Resten R⁸ substituiert sein können, und
A und B zusammen auch 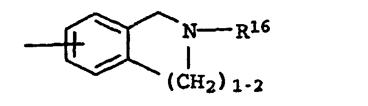 sein kann und R¹⁶ Wasserstoff, C₁-C₆-Alkyl und (CH₂)₁₋₄-Phenyl bedeutet, wobei der Phenyl-Ring noch mit maximal 2 Resten R⁶ substituiert sein kann, und
D -(CH₂)₀₋₂-O-(CH₂)₀₋₂, -(CH₂)ₘ-, CH=CH, -C≡C- sein kann und
R² Chlor, Brom, Fluor, C₁-C₆-Alkyl, NHCO-C₁-C₄-Alkyl, NHSO₂-C₁-C₄-Alkyl, NO₂, -O-C₁-C₄-Alkyl und NH₂-bedeutet, und
R³ -C₁-C₆-Alkyl, verzweigt oder unverzweigt, und der noch einen SCH₃-Rest, einen Phenyl-Ring, Imidazolyl-Ring, Indolyl-Ring und Cyclopentyl-, Cycloheptyl-, Cyclohexyl-Ring tragen kann, der seinerseits mit maximal zwei Resten R⁸ substituiert ist, wobei R⁸ Wasserstoff, C₁-C₄-Alkyl, verzweigt oder unverzweigt, -O-C₁-C₄-Alkyl, OH, Cl, F, Br, J, CF₃, NO₂, NH₂, CN, COOH, COO-C₁-C₄-Alkyl, NHCO-C₁-C₄-Alkyl, -NHSO₂-C₁-C₄-Alkyl und -SO₂-C₁-C₄-Alkyl bedeutet; und
Y Phenyl und Pyridin bedeutet und
R⁴ Wasserstoff, COOR⁹ und CO-Z bedeutet, worin Z NR¹⁰R¹¹, und 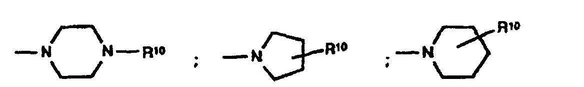 bedeutet,
R⁹ Wasserstoff, C₁-C₆-Alkyl, geradlinig oder verzweigt, bedeutet und das mit einem Phenylring substituiert kann, der selbst noch mit einem oder zwei Resten R¹² substituiert sein kann, und
R¹⁰ Wasserstoff, C₁-C₆-Alkyl, geradlinig oder verzweigt, bedeutet und das mit einem Phenylring substituiert kann, der selbst noch mit einem oder zwei Resten R¹² substituiert sein kann, und 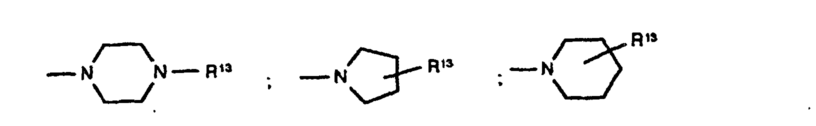 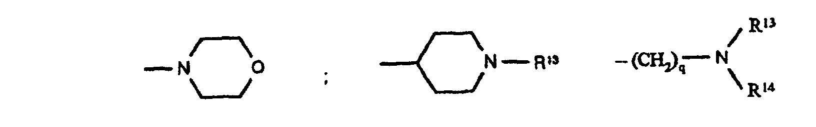
R¹¹ Wasserstoff, C₁-C₆-Alkyl, verzweigt oder unverzweigt, das noch mit einem Phenylring, der noch einen Rest R⁹ tragen kann, und substituiert sein kann, bedeutet, und
R¹² Wasserstoff, C₁-C₄-Alkyl, verzweigt oder unverzweigt, -O-C₁-C₄-Alkyl, OH, Cl, F, Br, J, CF₃, NO₂, NH₂, CN, COOH, COO-C₁-C₄-Alkyl, -NHCO-C₁-C₄-Alkyl, -NHCO-Phenyl,-NHSO₂-C₁-C₄-Alkyl, -NHSO₂-Phenyl, -SO₂-C₁-C₄-Alkyl und -SO₂-Phenyl bedeuten kann
R¹³ Wasserstoff, C₁-C₆-Alkyl, geradlinig oder verzweigt, bedeutet und das mit einem Phenylring substituiert sein kann, der selbst noch mit einem oder zwei Resten R¹² substituiert sein kann, und
R¹⁴ Wasserstoff, C₁-C₆-Alkyl, geradlinig oder verzweigt, bedeutet und das mit einem Phenylring substituiert sein kann, der selbst noch mit einem oder zwei Resten R¹² substituiert sein kann, und
n eine Zahl 0, 1 oder 2 bedeutet, und
m, q unabhängig voneinander eine Zahl 0, 1, 2, 3 oder 4 bedeutet.

2. Amide der Formel I gemäß dem Anspruch 1, wobei
A -CH₂-R¹
B Phenyl
D -CH=CH-
R² Wasserstoff
R³ Benzyl, CH₂-CH₃, CH₂-CH₂-CH₃, CH₂CH₂CH₂CH₃, CH₂CH₂CH₂CH₂CH₃ und
Y Phenyl und
R⁴ CO-NH₂ und
alle restlichen Variablen die gleiche Bedeutung wie im Anspruch 1 haben.

3. Amide der Formel I gemäß dem Anspruch 1, wobei
A -CH₂-R¹
B Phenyl
D -CH=CH-
R² Wasserstoff
R³ Benzyl, CH₂-CH₃, CH₂-CH₂-CH₃, CH₂CH₂CH₂CH₃, CH₂CH₂CH₂CH₂CH₃ und
Y Phenyl und
R⁴ Wasserstoff und
alle restlichen Variablen die gleiche Bedeutung wie im Anspruch 1 haben.

4. Amide der Formel I gemäß dem Anspruch 1, wobei
A -CH₂-R¹
B Phenyl
D -CH=CH-
R² Wasserstoff
R³ Benzyl, CH₂-CH₃, CH₂-CH₂-CH₃, CH₂CH₂CH₂CH₃, CH₂CH₂CH₂CH₂CH₃ und
Y Pyridin und
R⁴ Wasserstoff und
alle restlichen Variablen die gleiche Bedeutung wie im Anspruch 1 haben.

5. Amide der Formel I gemäß dem Anspruch 1, wobei
A -CH₂-R¹
B Phenyl
D -CH=CH
R² Wasserstoff
R³ Benzyl, CH₂-CH₃, CH₂-CH₂-CH₃, CH₂CH₂CH₂CH₃, CH₂CH₂CH₂CH₂CH₃ und
Y Pyridin und
R⁴ CONH₂ und
alle restlichen Variablen die gleiche Bedeutung wie im Anspruch 1 haben.

6. Verwendung von Amiden der Formel I gemäß dem Anspruch 1-5 zur Herstellung von Arzneimitteln zur Behandlung von Krankheiten.

7. Verwendung von Amiden der Formel I gemäß dem Anspruch 1-5 zur Herstellung von Arzneimitteln zur Inhibitierung von Cysteinproteasen.

8. Verwendung nach Anspruch 6 zur Herstellung von Arzneimitteln zur Inhibitorierung von Cystein1 proteasen wie Calpaine und Cathepsine, insbesondere Calpaine I und II und Cathepsine B und L.

9. Verwendung von Amiden der Formel I gemäß dem Anspruch 1-5 zur Herstellung als Arzneimittel zur Behandlung von Krankheiten, bei denen erhöhte Calpain-Aktivitäten auftreten.

10. Verwendung der Amiden der Formel I gemäß dem Anspruch 1-5 zur Herstellung von Arzneimitteln zur Behandlung von neurodegenerativen Krankheiten und neuronalen Schädigungen.

11. Verwendung nach Anspruch 9 zur Behandlung von solchen neurodegenerativen Krankheiten und neuronalen Schädigungen, die durch Ischämie, Trauma oder Massenblutungen ausgelöst werden.

12. Verwendung nach Anspruch 10 zur Behandlung von Himschlag und Schädel-Himtrauma.

13. Verwendung nach Anspruch 10 zur Behandlung von Alzheimerschen Krankheit und der Huntington-Krankheit.

14. Verwendung nach Anspruch 10 zur Behandlung von Epilepsien.

15. Verwendung der Verbindungen der Formel I gemäß dem Anspruch 1-5 zur Herstellung von Arzneimitteln zur Behandlung von Schädigungen des Herzens nach cardialen Ischämien, Schädigung durch Reperfusion nach Gefäßverschlüssen, Schädigungen der Nieren nach renalen Ischämien, Skelettmuskelschädigungen, Muskeldystrophien, Schädigungen, die durch Proliferation der glatten Muskelzellen entstehen, coronarer Vasospasmus, cerebraler Vasospasmus, Katarakten der Augen und Restenosis der Blutbahnen nach Angioplastie.

16. Verwendung der Amiden der Formel I gemäß dem Anspruch 1-5 zur Herstellung von Arzneimitteln zur Behandlung von Tumoren und deren Metastasierung.

17. Verwendung der Amiden der Formel I gemäß dem Anspruch 1-5 zur Herstellung von Arzneimitteln zur Behandlung von Krankheiten, bei denen erhöhte tnterleukin-I-Spiegel auftreten.

18. Verwendung der Amide gemäß Anspruch 1-5 zur Herstellung von Arzneimitteln zur Behandlung von immunologischen Krankheiten wie Entzündungen und rheumatische Erkrankungen.

19. Arzneimittelzubereitungen zur peroralen, parenteralen und intraperitonalen Anwendung, enthaltend pro Einzeldosis, neben den üblichen Arzneimittelhilfsstoffen, mindestens eines Amides I gemäß Anspruch 1-5.

## Claims

1. Amides having the general formula I 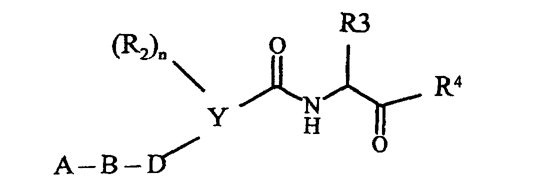 and their tautomeric forms, possible enantiomeric and diastereomeric forms, E and Z forms, and possible physiologically acceptable salts, wherein the variables have the following meaning:
A -(CH₂)ₚ-R¹, wherein R¹ can be pyrrolidine morpholine, hexahydroazepine, piperidine, -NR⁵R⁶ and 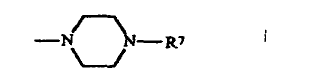 wherein the cyclic amines can also be substituted with one or two radicals R¹⁵ and R¹⁵ can denote hydrogen, C₁-C₆ alkyl, O-C₁-C₄ alkyl and phenyl and R⁵, R⁶ and R⁷ can mutually independently denote hydrogen, C₁-C₄ alkyl, cyclohexyl, cyclopentyl, CH₂Ph, Ph, CH₂CH₂Ph, wherein the phenyl rings can also be substituted with R⁶ and p can be 1 and 2 and
B can denote phenyl, pyridyl, pyrazyl, pyrimidyl and pyridazyl, wherein the rings can also be substituted with up to 2 radicals R⁸, and
A and B together can also be 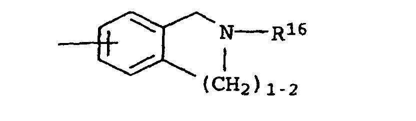 and R¹⁶ denotes hydrogen, C₁-C₆ alkyl and (CH₂)₁₋₄ phenyl, wherein the phenyl ring can also be substituted with a maximum of 2 radicals R⁶, and
D can be -(CH₂)₀₋₂-O-(CH₂)₀₋₂, -(CH₂)ₘ-, CH=CH, -C≡C-and
R² denotes chlorine, bromine, fluorine, C₁-C₆ alkyl, NHCO-C₁-C₄ alkyl, NHSO₂-C₁-C₄ alkyl, NO₂, -O-C₁-C₄ alkyl and NH₂, and
R³ denotes -C₁-C₆ alkyl, branched or unbranched, and which can also carry a SCH₃ radical, a phenyl ring, imidazolyl ring, indolyl ring and cyclopentyl, cycloheptyl, cyclohexyl ring, which in turn is substituted with a maximum of two radicals R⁸, wherein R⁸ denotes hydrogen, C₁-C₄ alkyl, branched or unbranched, -O-C₁-C₄ alkyl, OH, Cl, F, Br, I, CF₃, NO₂, NH₂, CN, COOH, COO-C₁-C₄ alkyl, NHCO-C₁-C₄ alkyl, -NHSO₂-C₁-C₄ alkyl and -SO₂-C₁-C₄ alkyl; and
Y denotes phenyl and pyridine and
R⁴ denotes hydrogen, COOR⁹ and CO-Z, wherein Z denotes NR¹⁰R¹¹, and 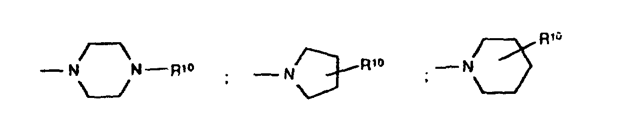
R⁹ denotes hydrogen, C₁-C₆ alkyl, straight or branched, and which can be substituted with a phenyl ring, which can itself also be substituted with one or two radicals R¹², and
R¹⁰ denotes hydrogen, C₁-C₆ alkyl, straight or branched, and which can be substituted with a phenyl ring, which can itself also be substituted with one or two radicals R¹², and 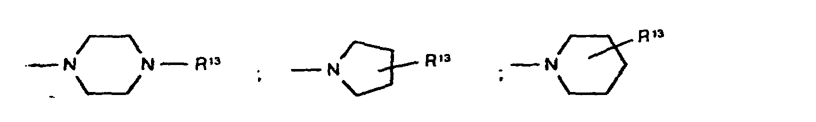 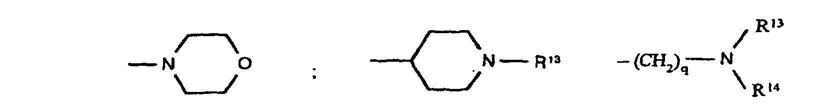
R¹¹ denotes hydrogen, C₁-C₆ alkyl, branched or unbranched, which can also be substituted with a phenyl ring, which can also carry a radical R⁹, and
R¹² can denote hydrogen, C₁-C₄ alkyl, branched or unbranched, -O-C₁-C₄ alkyl, OH, Cl, F, Br, I, CF₃, NO₂, NH₂, CN, COOH, COO-C₁-C₄ alkyl, -NHCO-C₁-C₄ alkyl, -NHCO phenyl, -NHSO₂-C₁-C₄ alkyl, -NNSO₂ phenyl, -SO₂-C₁-C₄ alkyl and -SO₂ phenyl
R¹³ denotes hydrogen, C₁-C₆ alkyl, straight or branched, and which can be substituted with a phenyl ring, which can itself also be substituted with one or two radicals R¹², and
R¹⁴ denotes hydrogen, C₁-C₆ alkyl, straight or branched, and which can be substituted with a phenyl ring, which can itself also be substituted with one or two radicals R¹², and
n denotes a number 0, 1 or 2, and
m,q mutually independently denote a number 0, 1, 2, 3 or 4.

2. Amides having formula I according to claim 1, wherein
A is -CH₂-R¹
B is phenyl
D is -CH=CH-
R² is hydrogen
R³ is benzyl, CH₂-CH₃, CH₂-CH₂-CH₃, CH₂CH₂CH₂CH₃, CH₂CH₂CH₂CH₂CH₃ and
Y is phenyl and
R⁴ is CO-NH₂ and
all other variables have the same meaning as in claim 1.

3. Amides having formula I according to claim 1, wherein
A is -CF₂-R¹
B is phenyl
D is -CH=CH-
R² is hydrogen
R³ is benzyl, CH₂-CH₃, CH₂-CH₂-CH₃, CH₂CH₂CH₂CH₃, CH₂CH₂CH₂CH₂CH₃ and
Y is phenyl and
R⁴ is hydrogen and
all other variables have the same meaning as in claim 1.

4. Amides having formula I according to claim 1, wherein
A is -CH₂-R¹
B is phenyl
D is -CH=CH-
R² is hydrogen
R³ is benzyl, CH₂-CH₃, CH₂-CH₂-CH₃, CH₂CH₂CK₂CH₃, CH₂CH₂CH₂CH₂CH₃ and
Y is pyridine and
R⁴ is hydrogen and
all other variables have the same meaning as in claim 1.

5. Amides having formula I according to claim 1, wherein
A is -CH₂-R¹
B is phenyl
D is -CH=CH
R² is hydrogen
R³ is benzyl, CH₂-CH₃, CH₂-CH₂-CH₃, CH₂CH₂CH₂CH₃, CH₂CH₂CH₂CH₂CH₃ and
Y is pyridine and
R⁴ is CONH₂ and
all other variables have the same meaning as in claim 1.

6. Use of amides having formula I according to claim 1 to 5 for the manufacture of medicaments for the treatment of diseases.

7. Use of amides having formula I according to claim 1 to 5 for the manufacture of medicaments for the inhibition of cysteine proteases.

8. Use according to claim 6 for the manufacture of medicaments for the inhibition of cysteine 1 proteases such as calpains and cathepsins, in particular calpains I and II and cathepains B and L.

9. Use of amides having formula I according to claim 1 to 5 for manufacture as medicaments for the treatment of diseases in which increased calpain activities occur.

10. Use of amides having formula I according to claim 1 to 5 for the manufacture of medicaments for the treatment of neurodegenerative diseases and neuronal damage.

11. Use according to claim 9 for the treatment of such neurodegenerative diseases and neuronal damage triggered by ischaemia, trauma or mass bleeding.

12. Use according to claim 10 for the treatment of stroke and craniocerebral trauma.

13. Use according to claim 10 for the treatment of Alzheimer's disease and Huntington's disease.

14. Use according to claim 10 for the treatment of epilepsies.

15. Use of compounds having formula I according to claim 1 to 5 for the manufacture of medicaments for the treatment of damage to the heart following cardial ischaemias, damage due to reperfusion following embolisms, damage to the kidneys following renal ischaemias, skeletal muscle damage, muscular dystrophies, damage arising from the proliferation of smooth muscle cells, coronary vasospasm, cerebral vasospasm, cataracts of the eye and reatenosis of the blood vessels following angioplasty.

16. Use of the amides having formula I according to claim 1 to 5 for the manufacture of medicaments for the treatment of tumours and metastaeising thereof.

17. Use of the amides having formula I according to claim 1 to 5 for the manufacture of medicaments for the treatment of diseases in which increased interleukin-1 levels occur.

18. Use of the amides according to claim 1 to 5 for the manufacture of medicaments for the treatment of immunological diseases such as inflammations and rheumatic diseases.

19. Pharmaceutical preparations for peroral, parenteral and intraperitonal administration, containing in each single dose, in addition to the conventional pharmaceutical auxiliary substances, at least one amide I according to claim 1 to 5.

## Revendications

1. Amides de formule générale I 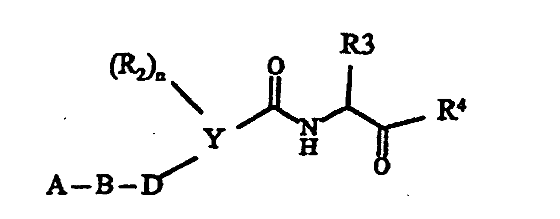 et leurs formes tautomères, leurs formes énantiomères et diastéréoisomères possibles, leurs formes E et Z, ainsi que leurs sels physiologiquement acceptables possibles, où les variables ont la signification suivante:
A peut représenter un reste -(CH₂)ₚ-R¹, où R¹ peut être un reste pyrrolidine, morpholine, hexahydroazépine, pipéridine, NR⁵R⁶ et 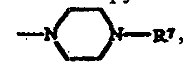 les amines cycliques pouvant être substituées en outre par un ou deux restes R¹⁵ et R¹⁵ représente l'hydrogène ou un reste alkyle en C₁-C₆, O-alkyle en C₁-C₆ ou phényle, et R⁵, R⁶ et R⁷ représentent indépendamment les uns des autres l'hydrogène ou un reste alkyle en C₁-C₄, cyclohexyle, cyclopentyle, CH₂Ph, Ph, CH₂CH₂Ph, les cycles phényle pouvant être substitués en outre par R⁶ et p peut être 1 ou 2, et
B peut représenter un reste phényle, pyridyle, pyrazyle, pyrimidyle ou pyridazyle, les cycles pouvant être substitués en outre par au plus 2 restes R⁸, et
A et B peuvent aussi représenter ensemble un reste 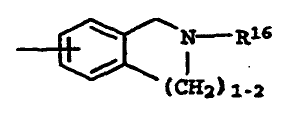 et R¹⁶ représente l'hydrogène ou un reste alkyle en C₁-C₆ ou (CH₂)₁₋₄-phényle, le cycle phényle pouvant être substitué en outre par au plus 2 restes R⁶, et
D peut être -(CH₂)₀₋₂-O-(CH₂)₀₋₂, -(CH₂)ₘ-, CH=CH, -C≡C-, et
R² représente le chlore, le brome, le fluor ou un reste alkyle en C₁-C₆, NHCO-(alkyle en C₁-C₄), NHSO₂-(alkyle en C₁-C₄), NO₂, -O-alkyle en C₁-C₄ ou NH₂, et
R³ représente un reste alkyle en C₁-C₆, linéaire ou ramifié, pouvant porter en outre un reste SCH₃, un cycle phényle, un cycle imidazolyle, un cycle indolyle ou un cycle cyclopentyle, cycloheptyle, cyclohexyle, qui peut lui-même être substitué par au plus 2 restes R⁸, R⁸ représentant l'hydrogène ou un reste alkyle en C₁-C₄ linéaire ou ramifié, -O-alkyle en C₁-C₄, OH, CI, F, Br, I, CF₃, NO₂, NH₂, CN, COOH, COO-(alkyle en C₁-C₄), NHCO-(alkyle en C₁-C₄), -NHSO₂-alkyle en C₁-C₄, ou -SO₂-alkyle en C₁-C₄; et
Y représente un reste phényle ou pyridine, et
R⁴ représente l'hydrogène ou un reste COOR⁹ ou CO-Z, où Z représente un reste NR¹⁰R¹¹ ou 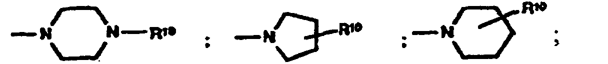
R⁹ représente l'hydrogène ou un reste alkyle en C₁-C₆ linéaire ou ramifié pouvant être substitué par un cycle phényle qui peut lui-même être substitué en outre par un ou deux restes R¹², et
R¹⁰ représente l'hydrogène ou un reste alkyle en C₁-C₆ linéaire ou ramifié pouvant être substitué par un cycle phényle qui peut lui-même être substitué en outre par un ou deux restes R¹², ou 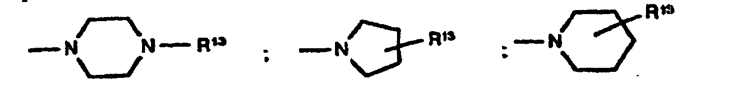 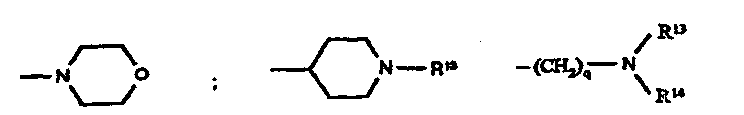
R¹¹ représente l'hydrogène ou un reste alkyle linéaire ou ramifié pouvant être substitué en outre par un cycle phényle qui peut porter en plus un reste R⁹, et
R¹² représente l'hydrogène ou un reste alkyle en C₁-C₄ linéaire ou ramifié, -O-alkyle en C₁-C₄, OH, Cl, F, Br, I, CF₃, NO₂, NH₂, CN, COOH, COO-(alkyle en C₁-C₄), NHCO-(alkyle en C₁-C₄), -NHCO-phényle, -NHSO₂-alkyle en C₁-C₄, -NHSO₂-phényle,-SO₂-alkyle en C₁-C₄ ou -SO₂-phényle,
R¹³ représente l'hydrogène ou un reste alkyle en C₁-C₆ linéaire ou ramifié pouvant être substitué par un cycle phényle qui peut lui-même être substitué en outre par un ou deux restes R¹², et
R¹⁴ représente l'hydrogène ou un reste alkyle en C₁-C₆ linéaire ou ramifié pouvant être substitué par un cycle phényle qui peut lui-même être substitué en outre par un ou deux restes R¹², et
n représente un nombre 0, 1 ou 2, et
m, q représentent, indépendamment l'un de l'autre, un nombre 0, 1, 2, 3, ou 4.

2. Amides de formule I selon la revendication 1, dans lesquels
A représente un reste -CH₂-R¹,
B représente un reste phényle,
D représente un reste -CH=CH-,
R² représente l'hydrogène,
R³ représente un reste benzyle, CH₂-CH₃, CH₂-CH₂-CH₃, CH₂CH₂CH₂CH₃, CH₂CH₂CH₂CH₂CH₃ et
Y représente un reste phényle et
R⁴ représente un reste CO-NH₂, et
toutes les autres variables ont la même signification que dans la revendication 1.

3. Amides de formule I selon la revendication 1, dans lesquels
A représente un reste -CH₂-R¹,
B représente un reste phényle,
D représente un reste -CH=CH-,
R² représente l'hydrogène,
R³ représente un reste benzyle, CH₂-CH₃, CH₂-CH₂-CH₃, CH₂CH₂CH₂CH₃, CH₂CH₂CH₂CH₂CH₃ et
Y représente un reste phényle et
R⁴ représente l'hydrogène, et
toutes les autres variables ont la même signification que dans la revendication 1.

4. Amides de formule I selon la revendication 1, dans lesquels
A représente un reste -CH₂-R¹,
B représente un reste phényle,
D représente un reste -CH=CH-,
R² représente l'hydrogène,
R³ représente un reste benzyle, CH₂-CH₃, CH₂-CH₂-CH₃, CH₂CH₂CH₂CH₃, CH₂CH₂CH₂CH₂CH₃ et
Y représente un reste pyridine et
R⁴ représente l'hydrogène, et
toutes les autres variables ont la même signification que dans la revendication 1.

5. Amides de formule I selon la revendication 1, dans lesquels
A représente un reste -CH₂-R¹,
B représente un reste phényle,
D représente un reste -CH=CH-,
R² représente l'hydrogène,
R³ représente un reste benzyle, CH₂-CH₃, CH₂-CH₂-CH₃, CH₂CH₂CH₂CH₃, CH₂CH₂CH₂CH₂CH₃ et
Y représente un reste pyridine et
R⁴ représente un reste CONH₂, et
toutes les autres variables ont la même signification que dans la revendication 1.

6. Utilisation d'amides de formule I selon la revendication 1-5 pour la préparation de médicaments destinés au traitement de maladies.

7. Utilisation d'amides de formule I selon la revendication 1-5 pour la préparation de médicaments destinés à inhiber les cystéine-protéases.

8. Utilisation selon la revendication 6 pour la préparation de médicaments destinés à inhiber des cystéine-1-protéases comme des calpaïnes et des cathepsines, en particulier les calpaïnes I et II et les cathepsines B et L.

9. Utilisation d'amides de formule I selon la revendication 1-5 pour la préparation de médicaments destinés au traitement de maladies dans lesquelles interviennent des activités élevées de calpaïne.

10. Utilisation d'amides de formule I selon la revendication 1-5 pour la préparation de médicaments destinés au traitement de maladies neurodégénératives et de lésions neuronales.

11. Utilisation selon la revendication 9 pour le traitement des maladies neurodégénératives et des lésions neuronales déclenchées par une ischémie, un traumatisme ou des hémorragies massives.

12. Utilisation selon la revendication 10, pour le traitement de l'attaque cérébrale et des traumatismes crânio-cérébraux.

13. Utilisation selon la revendication 10 pour le traitement de la maladie d'Alzheimer et de la maladie de Huntington.

14. Utilisation selon la revendication 10 pour le traitement d'épilepsies.

15. Utilisation des composés de formule I selon la revendication 1-5 pour la préparation de médicaments destinés au traitement de lésions cardiaques après des ischémies cardiales, de lésions de reperfusion après des occlusions vasculaires, de lésions rénales après des ischémies rénales, de lésions des muscles squelettiques, de dystrophies musculaires, de lésions dues à une prolifération des cellules des muscles lisses, de vasospasmes coronariens, de vasospasmes cérébraux, de cataractes des yeux et de la resténose des vaisseaux sanguins après une angioplastie.

16. Utilisation des amides de formule I selon la revendication 1-5 pour la préparation de médicaments destinés au traitement de tumeurs et de leurs métastases.

17. Utilisation des amides de formule I selon la revendication 1-5 pour la préparation de médicaments destinés au traitement de maladies dans lesquelles interviennent des niveaux élevés d'interleukine I.

18. Utilisation des amides de formule I selon la revendication 1-5 pour la préparation de médicaments destinés au traitement de maladies immunologiques comme des inflammations et des maladies rhumatismales.

19. Compositions de médicaments à utiliser par voie orale, parentérale et intrapéritonéale, contenant par dose unitaire, en plus des auxiliaires de médicaments habituels, au moins un amide I selon la revendication 1-5.
